# EUROPEAN PATENT SPECIFICATION

(11) **EP 3 823 953 B1**
(45) Date of publication and mention of the grant of the patent: **11.09.2024**
(21) Application number: 19773505.3
(22) Date of filing: 19.07.2019
(51) Int. Cl.: C07C 59/90, C07C 237/42, C07C 255/60, C07D 401/14

(54) **A NOVEL PROCESS FOR THE PREPARATION OF ANTHRANILIC DIAMIDES**
NEUARTIGES VERFAHREN ZUR HERSTELLUNG VON ANTHRANILDIAMIDEN
NOUVEAU PROCÉDÉ DE PRÉPARATION DE DIAMIDES ANTHRANILIQUES

(30) Priority: 20.07.2018 IN 201811027212
(43) Date of publication of application: 26.05.2021
(73) Proprietor: PI Industries Ltd., Udaipur, Rajasthan 313001 (IN)
(72) Inventor: KARRI, Phaneendrasai, Guntur, Andhra Pradesh 522006 (IN); PABBA, Jagadish, Udaipur, Rajasthan 313001 (IN); KALWAGHE, Amol Dnyaneshwar, Maharashtra 413708 (IN); SHINDE, Bharat Uttamrao, Maharashtra 423601 (IN); KLAUSENER, Alexander G.M., 50259 Pulheim (DE)
(74) Representative: Keltie LLP
(86) International application number: PCT/IB2019/056187
(87) International publication number: WO 2020/016841

(56) References cited:
- WO-A1-2011/009551
- DE-A1- 102006 032 168

## Description

### FIELD OF THE INVENTION:

The present invention relates to a novel process for the preparation of anthranilic diamides of the formula 1 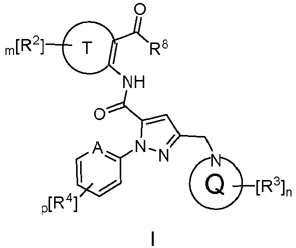
wherein, R², R³, R⁴, R⁸, m, n, p, A, Q and T are as defined in the description,
which can be used as insecticides.

### BACKGROUND OF THE INVENTION AND PROBLEM TO BE SOLVED

1-Heteroarylpyrazole-5-carboxylic acids are known to be important intermediates in the agrochemical industry, e. g. for the synthesis of anthranilic diamides which are useful to protect crops against harmful pests. Several methods have been disclosed in literature, by which these intermediates can be obtained. The formation of pyrazoles by the reaction of 1,3-dicarbonyls or of corresponding 1,3-bis-electrophilic compounds with hydrazines has been described in Synthesis 2004, pages 43-52. However, according to other references, namely WO2003016282, and Tetrahedron (2003), vol. 59, pages 2197-2205, the product formed in such reactions consists of a mixture of regioisomeric pyrazoles, meaning that the selectivity in the synthesis of the desired product is a challenge.

WO2007144100 describes a process for preparing tetrazolyl substituted N-(aryl or heteroaryl) pyrazole-5-carboxylic acid in which diisobutylaluminium hydride (DIBAL) or lithium aluminium hydride (LiAIH₄) is used. Typical shortcomings of this process are that it requires very low temperature conditions and that the use of DIBAL as a reagent is uneconomical.

WO2010069502 describes that tetrazolyl substituted anthranilic diamides can be prepared by reacting tetrazolyl substituted N-heteroaryl pyrazole acid with anthranilamides. It also describes that tetrazolyl substituted anthranilic diamides can be prepared by reacting tetrazolyl substituted benzoxazinones with appropriate amines. However, both the processes rarely offer good yield.

WO2010112178 describes a method for producing 1-pyridinylpyrazole-5-carboxylic acids or esters by the reaction of trihalo substituted acetylene ketones with pyridinyl hydrazines to first obtain an intermediate having a trihalomethyl substituent on the dihydro pyrazole ring, followed by dehydration and an oxidation step. However, trihalo substituted acetylene ketones are expensive starting materials which make this process economically unviable.

WO2011157664 and WO2013030100 disclose the following process for preparing tetrazolyl substituted 1-heteroarylpyrazole-5-carboxylic acid esters or 1-arylpyrazole-5-carboxylic acid esters:

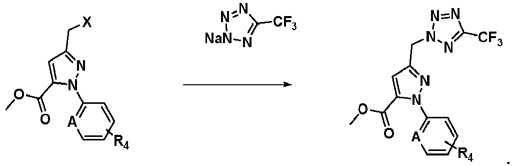

WO2013030100 additionally discloses a method for the preparation of tetrazolyl substituted anthranilic diamides from tetrazolyl substituted pyrazole carboxylic acids.

WO2011073101 describes a process for the preparation of 1-alkyl- or 1-aryl-substituted 5-pyrazolecarboxylic acids which involves the steps of converting substituted 1,3-dioxolanes or 1,4-dioxanes into 1-alkyl- or 1-aryl-substituted dihydro-1H-pyrazoles by reaction with alkyl or aryl hydrazines, and further converting said pyrazoles into 1-alkyl- or 1-aryl-substituted 5-pyrazole carboxylic acids.

Another prior art, DE102006032168 discloses the following two processes for the preparation of 1-heteroarylpyrazole-5-carboxylic acids as key intermediates: 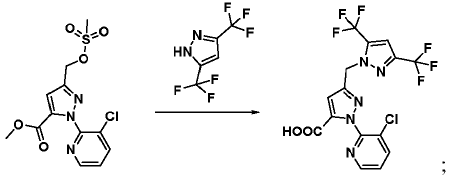 and

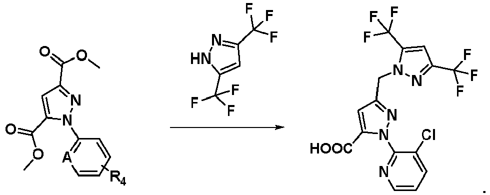

WO2011009551 describes a process for the preparation of 1-heteroaryl substituted pyrazole-5-carboxylic acids by reacting trihalo substituted alkoxy enones or enaminoketones with hydrazines to obtain 1-heteroaryl substituted dihydro-1H-pyrazoles as intermediates, followed by eliminating water and subsequent oxidation.

WO2013007604 describes a method for the preparation of tetrazolyl substituted anthranilic diamides by reacting pyrazole carboxylic acids with anthranilic esters, followed by hydrolysis to obtain acid intermediates which are then reacted with an appropriate amine.

An article published in SYNLETT 2005, No. 20, pp 3079-3082 discloses a method for the synthesis of useful intermediates as 1-phenyl-N-pyrazolylmethylpyrimidinones from 5-bromo-1,1,1-trichloro(fluoro)-4-methoxypent-3-en-2-ones with a moderate yield of 60-70%.

These processes described in the prior art have shortcomings such as poor yields of the desired intermediates or products, or synthetic procedures being not amenable to commercial scale, or of involving extreme reaction conditions or of lengthy reaction sequences making them uneconomical.

Thus, there is still a need for a process that obviates at least one shortcomings associated with the known processes.

### OBJECT AND SUMMARY OF THE INVENTION

It was therefore an objective to provide a novel and economically viable process for the preparation of anthranilic diamides of formula I.

Another objective was to provide novel intermediates for the preparation of anthranilic diamides of formula I.

Surprisingly, the present invention provides a solution to these objectives by offering a novel high yielding and economically attractive process that allows the preparation of anthranilic diamides and / or novel key intermediates to prepare such anthranilic diamides, overcoming at least one of the shortcomings of the processes described in the prior art.

These objectives were achieved according to the present invention by finding a process for preparing anthranilic diamides in which a compound of formula III 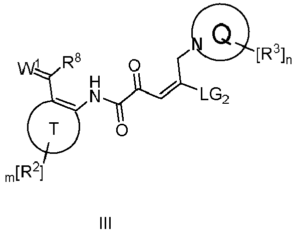
wherein, R², R³, R⁸, n, m, T, Q, W¹ and LG₂ are each as defined in the description,
is obtained by reacting a compound of formula V and a compound of formula IV 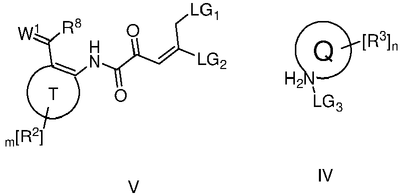
wherein, R², R³, R⁸, n, m, T, Q, W¹, LG₁, LG₂ and LG₃ are each as defined in the description.

The compound of formula V is obtained from a compound of formula VI 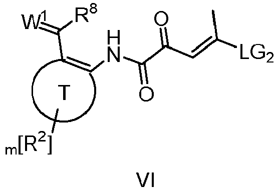
wherein, R², R⁸, m, T, W¹ and LG₂ are each as defined in the description,
by a halogenation reaction.

The compound of formula VI is obtained by reacting a compound of formula VII and a compound of formula VIII 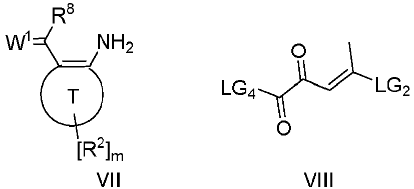 wherein, R², R⁸, m, T, W¹, LG₂ and LG₄ are each as defined in the description.

In an embodiment of the invention the compound of formula VIII is obtained by reacting a compound of formula IX and a compound of formula X 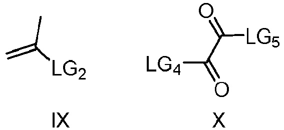 wherein, LG₂, LG₄ and LGs are each as defined in the description.

Finally, the compound of formula III is reacted with a hydrazine compound to obtain a compound of formula I.

### DETAILED DESCRIPTION OF THE INVENTION

### GENERAL DEFINITIONS

The definitions provided herein for the terminologies used in the present disclosure are for illustrative purpose only and in no manner limit the scope of the present invention disclosed in the present disclosure. As used herein, the terms "comprises", "comprising", "includes", "including", "has", "having", "contains", "containing", "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process or method.

The transitional phrase "consisting of" excludes any element, step or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of" is used to define a composition or method that includes materials, steps, features, components or elements, in addition to those literally disclosed, provided that these additional materials, steps, features, components or elements do not materially affect the basic and novel characteristic(s) of the claimed invention. The term "consisting essentially of" occupies a middle ground between "comprising" and "consisting of".

Further, unless expressly stated to the contrary, "or" refers to an inclusive "or" and not to an exclusive "or". For example, a condition A "or" B is satisfied by any one of the following: A is true (or present) and B is false (or not present), A is false (or not present) and B is true (or present), and both A and B are true (or present).

Also, the indefinite articles "a" and "an" preceding an element or component of the present invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

Compounds of the present disclosure may be present either in pure form or as mixtures of different possible isomeric forms such as stereoisomers or constitutional isomers. The various stereoisomers include enantiomers, diastereomers, chiral isomers, atropisomers, conformers, rotamers, tautomers, optical isomers, polymorphs, and geometric isomers. Any desired mixtures of these isomers fall within the scope of the claims of the present disclosure. One skilled in the art will appreciate that one stereoisomer may be more active and/or may exhibit beneficial effects when enriched relative to the other isomer(s) or when separated from the other isomer(s). Additionally, the person skilled in the art knows processes or methods or technology to separate, enrich, and/or to selectively prepare said isomers.

The meaning of various terms used in the description shall now be illustrated.

The term "alkyl", used either alone or in compound words such as "alkylthio" or "haloalkyl" or -N(alkyl) or alkylcarbonylalkyl or alkylsuphonylamino includes straight-chain or branched C₁ to C₂₄ alkyl, preferably C₁ to C₁₅ alkyl, more preferably C₁ to C₁₀ alkyl, most preferably C₁ to C₆ alkyl. Non-limiting examples of alkyl include methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and l-ethyl-2-methylpropyl or the different isomers. If the alkyl is at the end of a composite substituent, as, for example, in alkylcycloalkyl, the part of the composite substituent at the start, for example the cycloalkyl, may be mono- or polysubstituted identically or differently and independently by alkyl. The same also applies to composite substituents in which other radicals, for example alkenyl, alkynyl, hydroxyl, halogen, carbonyl, carbonyloxy and the like, are at the end.

The term "alkenyl", used either alone or in compound words includes straight-chain or branched C₂ to C₂₄ alkenes, preferably C₂ to C₁₅ alkenes, more preferably C₂ to C₁₀ alkenes, most preferably C₂ to C₆ alkenes. Non-limiting examples of alkenes include ethenyl, 1-propenyl, 2-propenyl, 1-methylethenyl, 1-butenyl, 2-butenyl, 3-butenyl, 1-methyl-1-propenyl, 2-methyl-l-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, l-methyl-2-butenyl, 2-methyl-2-butenyl, 3-methyl-2-butenyl, l-methyl-3-butenyl, 2-methyl-3-butenyl, 3-methyl-3-butenyl, 1,1-dimethyl-2-propenyl, 1,2-dimethyl-1-propenyl, 1,2-dimethyl-2 - propenyl, 1-ethyl-1-propenyl, l-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, l-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, l,l-dimethyl-3-butenyl, 1,2-dimethyl-l-butenyl, 1,2-dimethyl-2-butenyl, 1,2-dimethyl-3-butenyl, 1,3-dimethyl-1-butenyl, l,3-dimethyl-2-butenyl, l,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-l-butenyl, 3,3-dimethyl-2-butenyl, 1-ethyl-1-butenyl, 1-ethyl-2-butenyl, 1-ethyl-3-butenyl, 2-ethyl- 1-butenyl, 2-ethyl-2-butenyl, 2-ethyl-3-butenyl, l,l,2-trimethyl-2-propenyl, 1-ethyl-l-methyl-2-propenyl, l-ethyl-2-methyl-l-propenyl and l-ethyl-2-methyl-2-propenyl and the different isomers. "Alkenyl" also includes polyenes such as 1,2-propadienyl and 2,4-hexadienyl. This definition also applies to alkenyl as a part of a composite substituent, for example haloalkenyl and the like, unless defined specifically elsewhere.

Non-limiting examples of alkynes include ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, l-methyl-2-butynyl, 1-methyl-3-butynyl, 2-methyl-3-butynyl, 3-methyl-l-butynyl, 1,1-dimethyl-2-propynyl, 1-ethyl -2-propynyl, 1-hexynyl, 2-hexynyl, 3-hexynyl, 4-hexynyl, 5-hexynyl, 1-methyl-2-pentynyl, l-methyl-3-pentynyl, 1-methyl-4-pentynyl, 2-methyl-3-pentynyl, 2-methyl-4-pentynyl, 3-methyl-l-pentynyl, 3-methyl-4-pentynyl, 4-methyl-l-pentynyl, 4-methyl-2-pentynyl, 1,1-dimethyl-2-butynyl, l,l-dimethyl-3-butynyl, 1,2-dimethyl-3-butynyl, 2,2-dimethyl-3-butynyl, 3,3-dimethyl-l-butynyl, l-ethyl-2-butynyl, l-ethyl-3-butynyl, 2-ethyl-3-butynyl and 1-ethyl-l-methyl-2-propynyl and the different isomers. This definition also applies to alkynyl as a part of a composite substituent, for example haloalkynyl etc., unless specifically defined elsewhere. "Alkynyl" can also include moieties comprised of multiple triple bonds such as 2,5-hexadiynyl.

The term "cycloalkyl" means alkyl closed to form a ring. Non-limiting examples include cyclopropyl, cyclobutyl, cyclopentyl and cyclohexyl. This definition also applies to cycloalkyl as a part of a composite substituent, for example cycloalkylalkyl etc., unless specifically defined elsewhere.

Cycloalkenyl means alkenyl closed to form a ring including monocyclic, partially unsaturated hydrocarbyl groups. Non-limiting examples include cyclopentenyl and cyclohexenyl. This definition also applies to cycloalkenyl as a part of a composite substituent, for example cycloalkenylalkyl etc., unless specifically defined elsewhere.

Cycloalkynyl means alkynyl closed to form a ring including monocyclic, partially unsaturated groups. This definition also applies to cycloalkynyl as a part of a composite substituent, for example cycloalkynylalkyl etc., unless specifically defined elsewhere.

The term "cycloalkoxy", "cycloalkenyloxy" and the like are defined analogously. Non limiting examples of cycloalkoxy include cyclopropyloxy, cyclopentyloxy and cyclohexyloxy. This definition also applies to cycloalkoxy as a part of a composite substituent, for example cycloalkoxy alkyl etc., unless specifically defined elsewhere.

The term "halogen", either alone or in compound words such as "haloalkyl", includes F, Cl, Br or I. Further, when used in compound words such as "haloalkyl", said alkyl may be partially or fully substituted with halogen atoms which may be the same or different.

Non-limiting examples of "haloalkyl" include chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 1,1-dichloro-2,2,2-trifluoroethyl, and 1,1,1-trifluoroprop-2-yl. This definition also applies to haloalkyl as a part of a composite substituent, for example haloalkylaminoalkyl etc., unless specifically defined elsewhere.

The terms "haloalkenyl" and "haloalkynyl" are defined analogously except that, instead of alkyl groups, alkenyl and alkynyl groups are present as a part of the substituent.

The term "haloalkoxy" means straight-chain or branched alkoxy groups where some or all of the hydrogen atoms in these groups may be replaced by halogen atoms as specified above. Non-limiting examples of haloalkoxy include chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, pentafluoroethoxy and l,l,l-trifluoroprop-2-oxy. This definition also applies to haloalkoxy as a part of a composite substituent, for example haloalkoxyalkyl etc., unless specifically defined elsewhere.

The term "haloalkylthio" means straight-chain or branched alkylthio groups where some or all of the hydrogen atoms in these groups may be replaced by halogen atoms as specified above. Non-limiting examples of haloalkylthio include chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio, pentafluoroethylthio and l,l,l-trifluoroprop-2-ylthio. This definition also applies to haloalkylthio as a part of a composite substituent, for example haloalkylthioalkyl etc., unless specifically defined elsewhere.

Examples of "haloalkylsulfinyl" include CF₃S(O), CCl₃S(O), CF₃CH₂S(O) and CF₃CF₂S(O). Examples of "haloalkylsulfonyl" include CF₃S(O)₂, CCl₃S(O)₂, CF₃CH₂S(O)₂ and CF₃CF₂S(O)₂.

Hydroxy means -OH, Amino means -NRR, wherein R can be H or any possible substituent such as alkyl. Carbonyl means -C(O)- , carbonyloxy means -OC(O)-, sulfinyl means SO, sulfonyl means S(O)₂.

The term "alkoxy" used either alone or in compound words included C₁ to C₂₄ alkoxy, preferably C₁ to C₁₅ alkoxy, more preferably C₁ to C₁₀ alkoxy, most preferably C₁ to C₆ alkoxy. Examples of alkoxy include methoxy, ethoxy, propoxy, 1-methylethoxy, butoxy, 1-methylpropoxy, 2-methylpropoxy, 1,1-dimethylethoxy, pentoxy, 1-methylbutoxy, 2-methylbutoxy, 3-methylbutoxy, 2,2-dimethylpropoxy, 1-ethylpropoxy, hexoxy, 1,1-dimethylpropoxy, 1,2-dimethylpropoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and l-ethyl-2-methylpropoxy and the different isomers. This definition also applies to alkoxy as a part of a composite substituent, for example haloalkoxy, alkynylalkoxy, etc., unless specifically defined elsewhere.

"Alkoxyalkyl" denotes alkoxy substitution on alkyl. Non-limiting examples of "alkoxyalkyl" include CH₃OCH₂, CH₃OCH₂CH₂, CH₃CH₂OCH₂, CH₃CH₂CH₂CH₂OCH₂ and CH₃CH₂OCH₂CH₂.

The term "alkoxyalkoxy" denotes alkoxy substitution on alkoxy.

The term "alkylthio" includes branched or straight-chain alkylthio moieties such as methylthio, ethylthio, propylthio, 1-methylethylthio, butylthio, 1-methylpropylthio, 2-methylpropylthio, 1,1-dimethylethylthio, pentylthio, 1-methylbutylthio, 2-methylbutylthio, 3-methylbutylthio, 2,2-dimethylpropylthio, 1-ethylpropylthio, hexylthio, 1,1-dimethylpropylthio, 1,2-dimethylpropylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio and l-ethyl-2-methylpropylthio and the different isomers.

Halocycloalkyl, halocycloalkenyl, alkylcycloalkyl, cycloalkylalkyl, cycloalkoxyalkyl, alkylsulfinylalkyl, alkylsulfonylalkyl, haloalkylcarbonyl, cycloalkylcarbonyl, haloalkoxylalkyl, and the like, are defined analogously to the above examples.

The term "alkylthioalkyl" denotes alkylthio substitution on alkyl. Non-limiting examples of "alkylthioalkyl" include CH₂SCH₂, CH₂SCH₂CH₂, CH₃CH₂SCH₂, CH₃CH₂CH₂CH₂SCH₂ and CH₃CH₂SCH₂CH₂. "Alkylthioalkoxy" denotes alkylthio substitution on alkoxy. The term "cycloalkylalkylamino" denotes cycloalkyl substitution on alkyl amino.

The terms alkoxyalkoxyalkyl, alkylaminoalkyl, dialkylaminoalkyl, cycloalkylaminoalkyl, cycloalkylaminocarbonyl and the like, are defined analogously to "alkylthioalkyl" or cycloalkylalkylamino.

The term "alkoxycarbonyl" is an alkoxy group bonded to a skeleton via a carbonyl group (-CO-). This definition also applies to alkoxycarbonyl as a part of a composite substituent, for example cycloalkylalkoxycarbonyl and the like, unless specifically defined elsewhere.

The term "alkoxycarbonylalkylamino" denotes alkoxy carbonyl substitution on alkyl amino.

"Alkylcarbonylalkylamino" denotes alkyl carbonyl substitution on alkyl amino. The terms alkylthioalkoxycarbonyl, cycloalkylalkylaminoalkyl and the like are defined analogously.

Non-limiting examples of "alkylsulfinyl" include methylsulphinyl, ethylsulphinyl, propylsulphinyl, 1-methylethylsulphinyl, butylsulphinyl, 1-methylpropylsulphinyl, 2-methylpropylsulphinyl, 1,1-dimethylethylsulphinyl, pentylsulphinyl, 1-methylbutylsulphinyl, 2-methylbutylsulphinyl, 3-methylbutylsulphinyl, 2,2-dimethylpropylsulphinyl, 1-ethylpropylsulphinyl, hexylsulphinyl, 1,1-dimethylpropylsulphinyl, 1,2-dimethylpropylsulphinyl, 1-methylpentylsulphinyl, 2-methylpentylsulphinyl, 3-methylpentylsulphinyl, 4-methylpentylsulphinyl, 1,1-dimethylbutylsulphinyl, 1,2-dimethylbutylsulphinyl, 1,3-dimethylbutylsulphinyl, 2,2-dimethylbutylsulphinyl, 2,3-dimethylbutylsulphinyl, 3,3-dimethylbutylsulphinyl, 1-ethylbutylsulphinyl, 2-ethylbutylsulphinyl, 1,1,2-trimethylpropylsulphinyl, 1,2,2-trimethylpropylsulphinyl, 1-ethyl-1-methylpropylsulphinyl and 1-ethyl-2-methylpropylsulphinyl and the different isomers. The term "arylsulfinyl" includes Ar-S(O), wherein Ar can be any carbocyle or heterocylcle. This definition also applies to alkylsulphinyl as a part of a composite substituent, for example haloalkylsulphinyl etc., unless specifically defined elsewhere. Non-limiting examples of "alkylsulfonyl" include methylsulphonyl, ethylsulphonyl, propylsulphonyl, 1-methylethylsulphonyl, butylsulphonyl, 1-methylpropylsulphonyl, 2-methylpropylsulphonyl, 1,1-dimethylethylsulphonyl, pentylsulphonyl, 1-methylbutylsulphonyl, 2-methylbutylsulphonyl, 3-methylbutylsulphonyl, 2,2-dimethylpropylsulphonyl, 1-ethylpropylsulphonyl, hexylsulphonyl, 1,1-dimethylpropylsulphonyl, 1,2-dimethylpropylsulphonyl, 1-methylpentylsulphonyl, 2-methylpentylsulphonyl, 3-methylpentylsulphonyl, 4-methylpentylsulphonyl, 1,1-dimethylbutylsulphonyl, 1,2-dimethylbutylsulphonyl, 1,3-dimethylbutylsulphonyl, 2,2-dimethylbutylsulphonyl, 2,3-dimethylbutylsulphonyl, 3,3-dimethylbutylsulphonyl, 1-ethylbutylsulphonyl, 2-ethylbutylsulphonyl, 1,1,2-trimethylpropylsulphonyl, 1,2,2-trimethylpropylsulphonyl, 1-ethyl-1-methylpropylsulphonyl and 1-ethyl-2-methylpropylsulphonyl and the different isomers. The term "arylsulfonyl" includes Ar-S(O)₂, wherein Ar can be any carbocyle or heterocylcle. This definition also applies to alkylsulphonyl as a part of a composite substituent, for example alkylsulphonylalkyl etc., unless defined elsewhere.

"Alkylamino", "dialkylamino", and the like, are defined analogously to the above examples.

The term "carbocycle or carbocyclic" includes "aromatic carbocyclic ring system" and "nonaromatic carbocylic ring system" or polycyclic or bicyclic (spiro, fused, bridged, nonfused) ring compounds in which ring may be aromatic or non-aromatic (where aromatic indicates that the Hückel's rule is satisfied and non-aromatic indicates that the Hückel's rule is not statisfied).

The term "heterocycle" or "heterocyclic" includes "aromatic heterocycle" or "heteroaryl ring system" and "nonaromatic heterocycle ring system" or polycyclic or bicyclic (spiro, fused, bridged, non-fused) ring compounds in which ring may be aromatic or non-aromatic, wherein the heterocycle ring contains at least one heteroatom selected from N, O, S(O)₀₋₂, and or C ring member of the heterocycle may be replaced by C(=O), C(=S), C(=CR*R*) and C=NR*, * indicates integers.

The term "non-aromatic heterocycle" or "non-aromatic heterocyclic" means three- to fifteen-membered, preferably three- to twelve-membered, saturated or partially unsaturated heterocycle containing one to four heteroatoms from the group of oxygen, nitrogen and sulphur: mono, bi- or tricyclic heterocycles which contain, in addition to carbon ring members, one to three nitrogen atoms and/or one oxygen or sulphur atom or one or two oxygen and/or sulphur atoms; if the ring contains more than one oxygen atom, they are not directly adjacent; for example (but not limited to) oxetanyl, oxiranyl, aziridinyl, 2-tetrahydrofuranyl, 3-tetrahydrofuranyl, 2-tetrahydrothienyl, 3-tetrahydrothienyl, 1-pyrrolidinyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 1-pyrazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 1-imidazolidinyl, 2-imidazolidinyl, 4-imidazolidinyl, 1,2,4-oxadiazolidin-3-yl, l,2,4-oxadiazolidin-5-yl, l,2,4-thiadiazolidin-3-yl, 1,2,4-thiadiazolidin-5-yl, l,2,4-triazolidin-1-yl, l,2,4-triazolidin-3-yl, 1,3,4-oxadiazolidin-2-yl, l,3,4-thiadiazolidin-2-yl, 1,3,4-triazolidin-1-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, pyrrolinyl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-l-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-l-yl, 3,4-dihydropyrazol-3-yl, 3,4-dihydropyrazol-4-yl, 3,4-dihydropyrazol-5-yl, 4,5-dihydropyrazol-l-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, piperidinyl, 2-piperidinyl, 3-piperidinyl, 4-piperidinyl, pyrazynyl, morpholinyl, thiomorphlinyl, l,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl, l,2,4-hexahydrotriazin-3-yl, cycloserines, 2,3,4,5-tetrahydro[1H]azepin-1- or -2- or -3- or -4- or -5- or -6- or -7- yl, 3,4,5,6-tetra-hydro[2H]azepin-2- or -3- or -4- or -5- or -6- or-7-yl, 2,3,4,7-tetrahydro[1H]azepin-1- or -2- or -3- or -4- or -5- or -6- or-7- yl, 2,3,6,7-tetrahydro[1H]azepin-1- or -2- or -3- or -4- or -5- or -6- or -7- yl, hexahydroazepin-1- or -2- or -3- or -4- yl, tetra- and hexahydrooxepinyl such as 2,3,4,5-tetrahydro[1 H]oxepin-2- or -3- or -4- or -5- or -6- or -7- yl, 2,3,4,7-tetrahydro[1H]oxepin-2- or -3- or -4- or -5- or -6- or -7- yl, 2,3,6,7-tetrahydro[1H]oxepin-2- or -3- or -4- or -5- or -6- or -7- yl, hexahydroazepin-1- or -2- or -3- or -4- yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl. This definition also applies to heterocyclyl as a part of a composite substituent, for example heterocyclylalkyl etc., unless specifically defined elsewhere.

The term "heteroaryl" means 5 or 6-membered, fully unsaturated monocyclic ring system containing one to four heteroatoms from the group of oxygen, nitrogen and sulphur; if the ring contains more than one oxygen atom, they are not directly adjacent; 5-membered heteroaryl containing one to four nitrogen atoms or one to three nitrogen atoms and one sulphur or oxygen atom: 5-membered heteroaryl groups which, in addition to carbon atoms, may contain one to four nitrogen atoms or one to three nitrogen atoms and one sulphur or oxygen atom as ring members, for example (but not limited thereto) furyl, thienyl, pyrrolyl, isoxazolyl, isothiazolyl, pyrazolyl, oxazolyl, thiazolyl, imidazolyl, l,2,4-oxadiazolyl, l,2,4-thiadiazolyl, l,2,4-triazolyl, l,3,4-oxadiazolyl, l,3,4-thiadiazolyl, l,3,4-triazolyl, tetrazolyl; nitrogen-bonded 5-membered heteroaryl containing one to four nitrogen atoms, or benzofused nitrogen-bonded 5-membered heteroaryl containing one to three nitrogen atoms: 5-membered heteroaryl groups which, in addition to carbon atoms, may contain one to four nitrogen atoms or one to three nitrogen atoms as ring members and in which two adjacent carbon ring members or one nitrogen and one adjacent carbon ring member may be bridged by a buta-l,3-diene-l,4-diyl group in which one or two carbon atoms may be replaced by nitrogen atoms, where these rings are attached to the skeleton via one of the nitrogen ring members, for example (but not limited to) 1-pyrrolyl, 1-pyrazolyl, 1,2,4-triazol-l- yl, 1-imidazolyl, 1,2,3-triazol-l-yl and 1,3,4-triazol-l-yl.

6-membered heteroaryl which contains one to four nitrogen atoms: 6-membered heteroaryl groups which, in addition to carbon atoms, may contain, respectively, one to three and one to four nitrogen atoms as ring members, for example (but not limited thereto) 2-pyridinyl, 3-pyridinyl, 4-pyridinyl, 3-pyridazinyl, 4-pyridazinyl, 2-pyrimidinyl, 4-pyrimidinyl, 5-pyrimidinyl, 2-pyrazinyl, l,3,5-triazin-2-yl, l,2,4-triazin-3-yl and l,2,4,5-tetrazin-3-yl; benzofused 5-membered heteroaryl containing one to three nitrogen atoms or one nitrogen atom and one oxygen or sulphur atom: for example (but not limited to) indol-l-yl, indol-2-yl, indol-3-yl, indol-4-yl, indol-5-yl, indol-6-yl, indol-7-yl, benzimidazol-l-yl, benzimidazol-2-yl, benzimidazol-4-yl, benzimidazol-5-yl, indazol-l-yl, indazol-3-yl, indazol-4-yl, indazol-5-yl, indazol-6-yl, indazol-7-yl, indazol-2-yl, l-benzofuran-2-yl, l-benzofuran-3-yl, l-benzofuran-4-yl, l-benzofuran-5-yl, 1-benzofuran- 6-yl, l-benzofuran-7-yl, l-benzothiophen-2-yl, l-benzothiophen-3-yl, l-benzothiophen-4-yl, 1-benzothiophen-5-yl, l-benzothiophen-6-yl, l-benzothiophen-7-yl, l,3-benzothiazol-2-yl, 1,3- benzothiazol-4-yl, l,3-benzothiazol-5-yl, l,3-benzothiazol-6-yl, l,3-benzothiazol-7-yl, l,3-benzoxazol-2-yl, 1,3-benzoxazol-4-yl, l,3-benzoxazol-5-yl, 1,3-benzoxazol-6-yl and l,3-benzoxazol-7-yl; benzofused 6-membered heteroaryl which contains one to three nitrogen atoms: for example (but not limited to) quinolin-2-yl, quinolin-3-yl, quinolin-4-yl, quinolin-5-yl, quinolin-6-yl, quinolin-7-yl, quinolin-8-yl, isoquinolin-l-yl, isoquinolin-3-yl, isoquinolin-4-yl, isoquinolin-5-yl, isoquinolin-6-yl, isoquinolin-7-yl and isoquinolin-8-yl.

This definition also applies to heteroaryl as a part of a composite substituent, for example heteroarylalkyl etc., unless specifically defined elsewhere.

"Trialkylsilyl" includes 3 branched and/or straight-chain alkyl radicals attached to and linked through a silicon atom such as trimethylsilyl, triethylsilyl and t-butyl-dimethylsilyl. "Halotrialkylsilyl" denotes at least one of the three alkyl radicals is partially or fully substituted with halogen atoms which may be the same or different. "Alkoxytrialkylsilyl" denotes at least one of the three alkyl radicals is substituted with one or more alkoxy radicals which may be the same or different. "Trialkylsilyloxy" denotes a trialkylsilyl moiety attached through oxygen.

Non-limiting examples of "alkylcarbonyl" include C(O)CH₃, C(O)CH₂CH₂CH₃ and C(O)CH(CH₃)₂. Non-limiting examples of "alkoxycarbonyl" include CH₃OC(=O), CH₃CH₂OC(=O), CH₃CH₂CH₂OC(=O), (CH₃)₂CHOC(=O) and the different butoxy or pentoxycarbonyl isomers. Non-limiting examples of "alkylaminocarbonyl" include CH₃NHC(=O), CH₃CH₂NHC(=O), CH₃CH₂CH₂NHC(=O), (CH₃)₂CHNHC(=O) and the different butylamino -or pentylaminocarbonyl isomers. Non-limiting examples of "dialkylaminocarbonyl" include (CH₃)₂NC(=O), (CH₃CH₂)₂NC(=O), CH₃CH₂(CH₃)NC(=O), CH₃CH₂CH₂(CH₃)NC(=O) and (CH₃)₂CHN(CH₃)C(=O). Non-limiting examples of "alkoxyalkylcarbonyl" include CH₃OCH₂C(=O), CH₃OCH₂CH₂C(=O), CH₃CH₂OCH₂C(=O), CH₃CH₂CH₂CH₂OCH₂C(=O) and CH₃CH₂OCH₂CH₂C(=O). Non-limiting examples of "alkylthioalkylcarbonyl" include CH₃SCH₂C(=O), CH₃SCH₂CH₂C(=O), CH₃CH₂SCH₂C(=O), CH₃CH₂CH₂CH₂SCH₂C(=O) and CH₃CH₂SCH₂CH₂C(=O). The term haloalkylsufonylaminocarbonyl, alkylsulfonylaminocarbonyl, alkylthioalkoxycarbonyl, alkoxycarbonylalkyl amino and the like are defined analogously.

Non-limiting examples of "alkylaminoalkylcarbonyl" include CH₃NHCH₂C(=O), CH₃NHCH₂CH₂C(=O), CH₃CH₂NHCH₂C(=O), CH₃CH₂CH₂CH₂NHCH₂C(=O) and CH₃CH₂NHCH₂CH₂C(=O).

The term "amide" means A-R'C=ONR"-B, wherein R' and R" indicates substituents and A and B indicate any group.

The term "thioamide" means A-R'C=SNR"-B, wherein R' and R" indicates substituents and A and B indicate any group.

The total number of carbon atoms in a substituent group is indicated by the "Cᵢ-Cⱼ" prefix where i and j are numbers from 1 to 21. For example, C₁-C₃ alkylsulfonyl designates methylsulfonyl through propylsulfonyl; C₂ alkoxyalkyl designates CH₃OCH₂; C₃ alkoxyalkyl designates, for example, CH₃CH(OCH₃), CH₃OCH₂CH₂ or CH₃CH₂OCH₂; and C₄ alkoxyalkyl designates the various isomers of an alkyl group substituted with an alkoxy group containing a total of four carbon atoms, examples including CH₃CH₂CH₂OCH₂ and CH₃CH₂OCH₂CH₂. In the above recitations, when a compound of Formula I is comprised of one or more heterocyclic rings, all substituents are attached to these rings through any available carbon or nitrogen by replacement of a hydrogen on said carbon or nitrogen. When a compound is substituted with a substituent bearing a subscript that indicates the number of said substituents can exceed 1, said substituents (when they exceed 1) are independently selected from the group of defined substituents. Further, when the subscript m in (R)ₘ indicates an integer ranging from for example 0 to 4 then the number of substituents may be selected from the integers between 0 and 4 inclusive.

When a group contains a substituent which can be hydrogen, then, when this substituent is taken as hydrogen, it is recognized that said group is being un-substituted.

The embodiments herein and the various features and advantageous details thereof are explained with reference to the non-limiting embodiments in the description. Descriptions of well-known components and processing techniques are omitted so as to not unnecessarily obscure the embodiments herein. The examples used herein are intended merely to facilitate an understanding of ways in which the embodiments herein may be practiced and to further enable those of skilled in the art to practice the embodiments herein. Accordingly, the examples should not be construed as limiting the scope of the embodiments herein.

The description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the scope of the embodiments as described herein.

Any discussion of documents, acts, materials, devices, articles and the like that has been included in this specification is solely for the purpose of providing a context for the disclosure. It is not to be taken as an admission that any or all of these matters form a part of the prior art base or were common general knowledge in the field relevant to the disclosure as it existed anywhere before the priority date of this application.

The compounds synthesized by the novel and inventive process of the present invention may, if appropriate, be present as mixtures of different possible isomeric forms, especially of stereoisomers, for example E and Z, threo and erythro, and also optical isomers, but if appropriate also of tautomers. Both the E and the Z isomers, and also the threo and erythro isomers, and the optical isomers, any desired mixtures of these isomers and the possible tautomeric forms are disclosed and claimed.

The present invention relates to a novel process for preparing a compound of formula I; 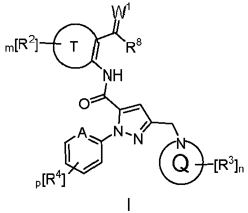 wherein,
R⁸ is selected from the group consisting of hydrogen, hydroxy, chlorine, bromine, iodine, C₁-C₆-alkoxy, C₃-C₈-cycloalkoxy or NR^{1a}R^{1b};
   wherein,
   R^{1a} and R^{1b} are independently selected from the group consisting of hydrogen, C₁-C₆-alkyl, C₁-C₆-haloalkyl, C₃-C₆-cycloalkyl, (C₁-C₆-alkyl)-C₃-C₆-cycloalkyl and (C₃-C₆-cycloalkyl)-C₁-C₆-alkyl, or
   R^{1a} and R^{1b} together with the N atom to which they are attached form N=S(=O)₀₋₂(C₁-C₆-alkyl)₂ or 3- or 4- or 5- or 6- membered heterocyclic ring comprising one or more heteroatoms selected from N, O and S; and the C atom of the heterocyclic ring may be replaced with C(=O), C(=S) and C(=NR⁷),
      wherein,
      the heterocyclic ring may be substituted with at least group selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, C₁-C₆-haloalkylsulphonyl, and
      R⁷ is selected from the group consisting of hydrogen, hydroxy, cyano, C₁-C₆-alkyl, C₁-C₆-haloalkyl, C₁-C₆-alkoxy, C₁-C₆-haloalkoxy, C₃-C₆-cycloalkyl, C₃-C₆-halocycloalkyl, C₃-C₆-cycloalkoxy, C₃-C₆-halocycloalkoxy;
W¹ is selected from O, S or NR⁵,
   wherein, R⁵ is selected from the group consisting of hydrogen, hydroxy, cyano, nitro, halogen, C₁-C₆-alkyl, C₁-C₆-haloalkyl, C₁-C₆-alkoxy, C₁-C₆-haloalkoxy, C₃-C₆-cycloalkyl, C₃-C₆-halocycloalkyl;
   or
   W¹ and R⁸ together with the carbon atom to which they are attached may form an oxazoline ring 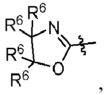 wherein, 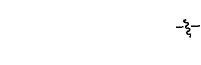
   indicates the point of attachment to T, and
   R⁶ is independently hydrogen or C₁-C₆-alkyl;
aryl or a heteroaryl ring or ring system;
R² is selected from the group consisting of hydrogen, halogen, cyano, nitro, C₁-C₆-alkyl, C₁-C₆-haloalkyl and C₃-C₆-cycloalkyl;
A is N or C;
R³ is selected from the group consisting of hydrogen, halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl and C₁-C₆-haloalkylsulphonyl;
R⁴ is selected from the group consisting of hydrogen, halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, C₁-C₆-haloalkylsulphonyl or NR^{a}R^{b};
   wherein, R^{a} and R^{b} are independently selected from the group consisting of hydrogen, C₁-C₆-alkyl, C₃-C₆-cycloalkyl or R^{a} and R^{b} together with the N atom to which they are attached form a substituted or unsubstituted 3- to 6- membered heterocylic ring,
   wherein, the substitution on 3- to 6- membered heterocyclic ring is selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl and C₁-C₆-haloalkylsulphonyl;
Q is a 3-, 4- or 5 membered heterocyclic ring;
n is an integer 0 to 4;
m is an integer 0 to 6; and
p is an integer 0 to 5.

In one of the particular embodiments the present invention relates to a novel process for preparing a compound of formula I;
wherein,
R⁸ is selected from the group consisting of hydroxy, chlorine, C₁-C₆-alkoxy or NR^{1a}R^{1b}, wherein, R^{1a} and R^{1b} are independently selected from the group consisting of hydrogen, C₁-C₆-alkyl, (C₁-C₆-alkyl)-C₃-C₆-cycloalkyl and (C₃-C₆-cycloalkyl)-C₁-C₆-alkyl;
W¹ is O; T is phenyl; R² is selected from the group consisting of hydrogen, halogen, cyano and C₁-C₆-alkyl; A is N; R³ is selected from the group consisting of hydrogen, halogen and C₁-C₆-haloalkyl; R⁴ is selected from the group consisting of hydrogen or halogen; Q is a 5 membered heterocyclic ring; n is an integer 1; m is an integer 2; p is an integer 1 or 2.

In another particular embodiment the present invention relates to a novel process for preparing a compound of formula I;
wherein,
R⁸ is NR^{1a}R^{1b}, wherein, R^{1a} is hydrogen; and R^{1b} is C₁-C₆-alkyl, (C₁-C₆-alkyl)-C₃-C₆-cycloalkyl and (C₃-C₆-cycloalkyl)-C₁-C₆-alkyl; W¹ is O; T is phenyl; R² is selected from the group consisting of halogen, cyano and C₁-C₆-alkyl; A is N; R³ is C₁-C₆-haloalkyl; R⁴ is halogen; Q is a tetrazolyl ring; n is an integer 1; m is an integer 2; p is an integer 1.

In yet another particular embodiment the present invention relates to a novel process for preparing a compound of formula I;
wherein,
R⁸ is NR^{1a}R^{1b}, wherein, R^{1a} is hydrogen; and R^{1b} is methyl or 1-cyclopropylethyl; W¹ is O; T is phenyl; R² is 4-cyano and 2-methyl, or 2-bromo and 4-chloro; A is N; R³ is 5-trifluoro methyl; R⁴ is 3-chlorine; Q is a 2-tetrazolyl ring; n is an integer 1; m is an integer 2; p is an integer 1.

The process for preparing the compounds of formula I is described herein after.

Process **step** (a): In the step (a) of the process, a compound of formula VIII and a compound of formula VII are reacted in a suitable solvent and optionally using a suitable coupling reagent to obtain a compound of formula VI; 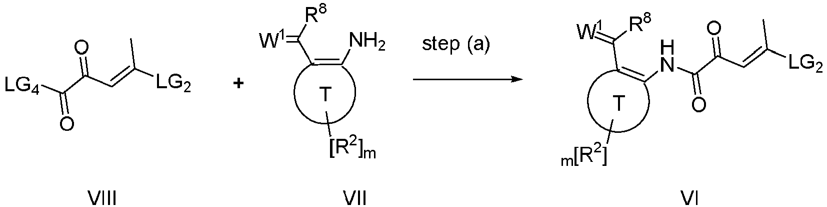 wherein,
LG₂ is selected from the group consisting of fluorine, chlorine, bromine, iodine or OR¹²,
   wherein, R¹² is selected from the group consisting of hydrogen, substituted or unsubstituted C₁-C₆-alkyl, substituted or unsubstituted aryl-C₁-C₆-alkyl, substituted or unsubstituted aryl, -(C=O)-C₁-C₆-alkyl, -(C=O)-C₁-C₆-haloalkyl, -(C=O)O-C₁-C₆-alkyl, -(C=O)O-haloC₁-C₆-alkyl, SO₂-C₁-C₆-alkyl, SO₂-C₁-C₆-haloalkyl, substituted or unsubstituted SO₂-aryl and alkylthio;
   LG₄ is selected from the group consisting of chlorine, bromine, iodine, N(OR¹³)R¹³, OR¹³ or 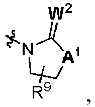 wherein, R¹³ is selected from the group consisting of hydrogen, substituted or unsubstituted C₁-C₆-alkyl, substituted or unsubstituted aryl-C₁-C₆-alkyl, substituted or unsubstituted aryl, -(C=O)-C₁-C₆-alkyl, -(C=O)-C₁-C₆-haloalkyl, -(C=O)O-C₁-C₆-alkyl, - (C=O)O-haloC₁-C₆-alkyl, SO₂-C₁-C₆-alkyl, SO₂-C₁-C₆-haloalkyl, substituted or unsubstituted SO₂-aryl and alkylthio,
      wherein, the substitutions on C₁-C₆-alkyl, aryl-C₁-C₆-alkyl, aryl and SO₂-aryl of R¹² and R¹³ are independently selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl and C₁-C₆-haloalkylsulphonyl,
W² and A¹ are independently selected from O, S or NR⁵, and
   R⁹ is selected from the group consisting of hydrogen, halogen, cyano, C₁-C₆-alkyl, C₁-C₆-haloalkyl, C₁-C₆-alkoxy, C₁-C₆-haloalkoxy, C₃-C₆-cycloalkyl, C₃-C₆-halocycloalkyl, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, aryl and arylalkyl;
R², R⁵, R⁸, m, T, and W¹ are each as defined herein above.

In one embodiment the compound of formula VI may be isolated.

In another embodiment the compound of formula VI may not be isolated.

The suitable solvent useful for the purpose of process step (a) is selected from the group consisting of aliphatic, alicyclic or aromatic hydrocarbons, for example petroleum ether, n-hexane, n-heptane, cyclohexane, methylcyclohexane, benzene, toluene, xylene or decalin; halogenated hydrocarbons, for example chlorobenzene, dichlorobenzene, dichloromethane, chloroform, tetrachloromethane, dichloroethane or trichloroethane; ethers such as diethyl ether, diisopropyl ether, methyl tert-butyl ether, methyl tert-amyl ether, dioxane, tetrahydrofuran, 2-methyl tetrahydrofuran, 1,2-dimethoxyethane, 1,2-diethoxyethane, cyclopentylmethylether or anisole; nitriles such as acetonitrile, propionitrile, n- or isobutyronitrile or benzonitrile; amides such as N,N-dimethylformamide, N,N-dimethylacetamide, N-methylformanilide, N-methylpyrrolidone or hexanlethylphosphoranlide; sulphoxides such as dimethyl sulphoxide; sulphones such as sulpholane; alcohols such as methanol, ethanol, or isopropanol.

For coupling of the compound of formula VIII and the compound of formula VII leading to the formation of the compound of formula VI the following coupling reagents are useful: pyridine, 1- or 2- or 3-picoline, triethyl amine, *N,N*-diisopropylethylamine, 2,6-di-tert-butylpyridine, 1,5-diazabicyclo(4.3.0)non-5-ene, 1,8-diazabicycloundec-7-ene, lithium diisopropylamide, sodium bis(trimethylsilyl)amide, and potassium bis(trimethylsilyl)amide or combinations thereof.

The formation of the compound of formula VI is carried out within a temperature range of from 0 to 120 °C.

The reaction time is not critical and depends on the batch size, temperature, reagent and solvent employed. Typically, the reaction time may vary from few minutes to several hours.

The process step (a) is usually performed under standard pressure conditions. It is, however, also possible to perform the process step (a) under reduced pressure or elevated pressure conditions.

The compound of formula VIII, wherein LG₄ is N(OR¹³)R¹³, OR¹³, or 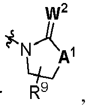 is obtained by the processes known in the literature, for example, Synthesis (2013), 45(7), 925-930.

In one embodiment, the compound of formula VIII is obtained by reacting a compound of formula X with a compound of formula IX in a suitable solvent and optionally a suitable reagent; 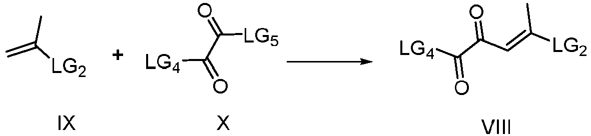 wherein, LG₅ is hydroxy or halogen; and LG₂ and LG₄ are each as defined herein above.

The suitable solvent useful for the purpose preparing the compound of formula VIII is selected from the group consisting of aliphatic, alicyclic or aromatic hydrocarbons, for example petroleum ether, n-hexane, n-heptane, cyclohexane, methylcyclohexane, benzene, toluene, xylene or decalin; halogenated hydrocarbons, for example chlorobenzene, dichlorobenzene, dichloromethane, chloroform, tetrachloromethane, dichloroethane or trichloroethane; ethers such as diethyl ether, diisopropyl ether, methyl tert-butyl ether, methyl tert-amyl ether, dioxane, tetrahydrofuran, 2-methyl tetrahydrofuran, 1,2-dimethoxyethane, 1,2-diethoxyethane, cyclopentylmethylether or anisole; nitriles such as acetonitrile, propionitrile, n- or isobutyronitrile or benzonitrile; amides such as N,N-dimethylformamide, N,N-dimethylacetamide, N-methylformanilide, N-methylpyrrolidone or hexanlethylphosphoranlide; sulphoxides such as dimethyl sulphoxide; sulphones such as sulpholane; alcohols such as methanol, ethanol, or isopropanol.

The suitable reagent useful in obtaining the compound of formula VIII is selected from the group consisting of pyridine, 1- or 2- or 3-picoline, triethyl amine, *N,N*-diisopropylethylamine, 2,6-di-tert-butylpyridine, 1,5-diazabicyclo(4.3.0)non-5-ene, 1,8-diazabicycloundec-7-ene, lithium diisopropylamide, sodium bis(trimethylsilyl)amide, and potassium bis(trimethylsilyl)amide or combinations thereof.

The formation of the compound of formula VIII is carried out within a temperature range of from 0 to 120 °C.

The reaction time is not critical and depends on the batch size, temperature, reagent and solvent employed. Typically, the reaction time may vary from few minutes to several hours.

The process for the formation of the compound of formula VIII is usually performed under standard pressure conditions. It is, however, also possible to perform the formation of the compound of formula VIII under reduced pressure or elevated pressure conditions.

In one embodiment the compound of formula VIII may be isolated.

In one embodiment the compound of formula VIII may not be isolated.

**Process step (b):** In the process step (b), the compound of formula VI is halogenated into a compound of formula V by using a suitable halogenating agent in a suitable solvent; 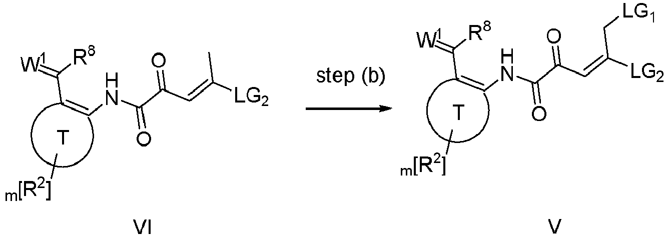 wherein, LG₁ is selected from the group consisting of chlorine, bromine or iodine; and R², R⁸, m, T, W¹ and LG₂ are each as defined herein above.

The suitable halogenating agent is selected from the group consisting of N-halosuccinimide, X₂, N-halosaccharine and halohydantoine.

N-halosuccinimide is selected from the group consisting of N-chlorosuccinimide, N-bromosuccinimide and N-iodosuccinimide. Preferably, N-halosuccinimide is N-chlorosuccinimide and N-bromosuccinimide.

X₂ is selected from the group consisting of Cl₂, Br₂ or I₂.

N-halosaccharine is selected from the group consisting of N-chlorosaccharine, N-bromosaccharine and N-iodosaccharine. Preferably, N-halosaccharine is N-chlorosaccharine and N-bromosaccharine.

N-halohydantoine is selected from the group consisting of N-chlorohydantoine, N-bromohydantoine and N-iodohydantoine. Preferably, N-halohydantoine is N-chlorohydantoine and N-bromohydantoine.

The halogenation reaction can be accelerated by the addition of radical initiators such as dibenzoyl peroxide, hydrogen peroxide, di(n-propyl)peroxydicarbonate, t-butyl peroxybenzoate, methyl ethyl ketone peroxide, 2,5-dimethyl-2,5-di(t-butylperoxy)-3-hexyne, di(t-butyl)peroxide, acetone peroxide, dicumyl peroxide, azobisisobutyronitrile, bis(2-ethylhexyl)peroxydicarbonate, (peroxybis(propane-2,2-diyl))dibenzene, peracetic acid, metachloroperbenzoic acid, Payne's reagent, magnesium monoperphthalate, trifluroperacetic acid, trichloroperacetic acid, 2, 4-dinitorperbenzoic acid, caro's acid and potassium caroate.

The halogenation reaction when carried out using X₂ as halogenating agent then it can be accelerated by using a source of light *hv.*

The suitable solvent useful for the purpose halogenation of the compound of formula VI is selected from the group consisting of aliphatic, alicyclic or aromatic hydrocarbons, for example petroleum ether, n-hexane, n-heptane, cyclohexane, methylcyclohexane, benzene, toluene, xylene or decalin; halogenated hydrocarbons, for example chlorobenzene, dichlorobenzene, dichloromethane, chloroform, tetrachloromethane, dichloroethane or trichloroethane; ethers such as diethyl ether, diisopropyl ether, methyl tert-butyl ether, methyl tert-amyl ether, dioxane, tetrahydrofuran, 2-methyl tetrahydrofuran, 1,2-dimethoxyethane, 1,2-diethoxyethane, cyclopentylmethylether or anisole; nitriles such as acetonitrile, propionitrile, n- or isobutyronitrile or benzonitrile; amides such as N,N-dimethylformamide, N,N-dimethylacetamide, N-methylformanilide, N-methylpyrrolidone or hexanlethylphosphoranlide; sulphoxides such as dimethyl sulphoxide; sulphones such as sulpholane; alcohols such as methanol, ethanol, or isopropanol.

The halogenation reaction is carried out within a temperature range of from 0 to 120 °C.

The reaction time is not critical and depends on the batch size, temperature, reagent and solvent employed. Typically, the reaction time may vary from few minutes to several hours.

The halogenation reaction is usually performed under standard pressure conditions. It is, however, also possible to perform the halogenation reaction under reduced pressure or elevated pressure conditions.

In one embodiment the compound of formula V may be isolated.

In one embodiment the compound of formula V may not be isolated.

Process step (c): In the process step (c), the compound of formula V is reacted with a compound of formula IV in a suitable solvent to obtain a compound of formula III; 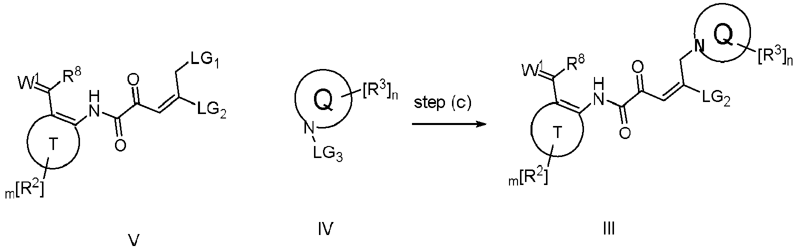
LG₃ is selected from the group consisting of hydrogen, halogen, Si(R¹¹)₃ and alkali metal selected from sodium, potassium and lithium,
   wherein, R¹¹ is selected from the group consisting of hydrogen, halogen, hydroxy, C₁-C₆-alkyl, C₁-C₆-haloalkyl, C₃-C₆-cycloalkyl, C₃-C₆-halocycloalkyl, C₁-C₆-alkoxy and C₁-C₆-haloalkoxy;
R², R³, R⁸, m, n, Q, T, W¹, LG₁ and LG₂ are each as defined herein above.

The suitable solvent useful for the purpose substituting LG₁ with the compound of formula IV leading to the formation of the compound of formula III is selected from the group consisting of aliphatic, alicyclic or aromatic hydrocarbons, for example petroleum ether, n-hexane, n-heptane, cyclohexane, methylcyclohexane, benzene, toluene, xylene or decalin; halogenated hydrocarbons, for example chlorobenzene, dichlorobenzene, dichloromethane, chloroform, tetrachloromethane, dichloroethane or trichloroethane; ethers such as diethyl ether, diisopropyl ether, methyl tert-butyl ether, methyl tert-amyl ether, dioxane, tetrahydrofuran, 2-methyl tetrahydrofuran, 1,2-dimethoxyethane, 1,2-diethoxyethane, cyclopentylmethylether or anisole; nitriles such as acetonitrile, propionitrile, n- or isobutyronitrile or benzonitrile; amides such as N,N-dimethylformamide, N,N-dimethylacetamide, N-methylformanilide, N-methylpyrrolidone or hexanlethylphosphoranlide; sulphoxides such as dimethyl sulphoxide; sulphones such as sulpholane; alcohols such as methanol, ethanol, or isopropanol.

The formation of the compound of formula III is carried out within a temperature range of from 15 to 150 °C.

The reaction time is not critical and depends on the batch size, temperature, reagent and solvent employed. Typically, the reaction time may vary from few minutes to several hours.

The formation of the compound of formula III is usually performed under standard pressure conditions. It is, however, also possible to perform the halogenation reaction under reduced pressure or elevated pressure conditions.

In one embodiment the compound of formula III may be isolated.

In one embodiment the compound of formula III may not be isolated.

It has been found that the replacement of LG₁ by the compound of formula IV in the process step (d) is also critical to the present invention as none of the prior art discloses or suggests to coupling of the compound of formula V and the compound of formula IV.

It is also found that the reaction is highly regioselective. For example, when Q is tetrazolyl ring, R³ is trifluoro methyl, LG₁ is halogen, LG₂ is OMe and n is an integer 1, the product III-1 is formed in higher percentage than that of the product III-2: 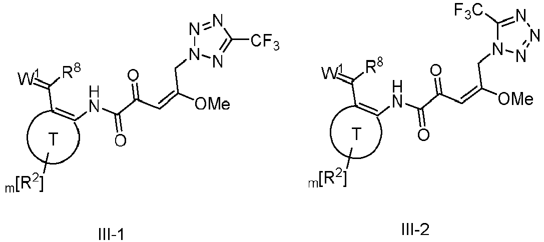 wherein, R², R⁸, W¹, and T are as defined herein above.

**Process step (d):** In the process step (d), the compound of formula III is reacted with a compound of formula II in a suitable solevent and using a suitable dehydrating agent to obtain the compound of formula I; 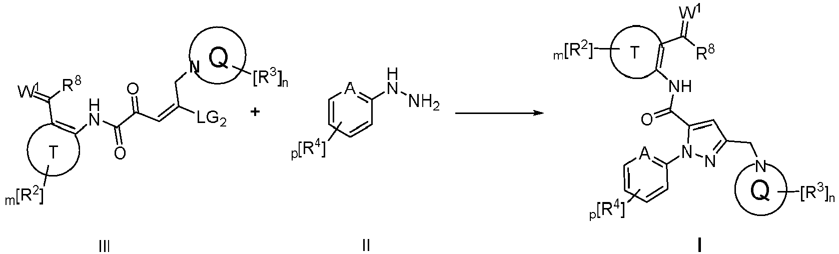 wherein, R², R³, R⁴, R⁸, m, n, p, A, Q, T, W¹ and LG₂ are each as defined herein above.

Particularly, the substituent LG₂ in the compounds of formulae VIII, VI, V, and III is selected from the group consisting of chlorine, bromine or OR¹², wherein, R¹² is C₁-C₆-alkyl.

More particularly, the substituent LG₂ in the compounds of formulae VIII, VI, V, and III is OR¹², wherein, R¹² is C₁-C₃-alkyl.

Most particularly, the substituent LG₂ in the compounds of formulae VIII, VI, V, and III is methoxy.

Particularly, the substituent LG₄ in the compound of formula VIII is selected from the group consisting of chlorine or OR¹³, wherein, R¹³ is selected from the group consisting of hydrogen or C₁-C₆-alkyl.

More particularly, the substituent LG₄ in the compound of formula VIII is chlorine.

Particularly, the substituent LG₁ in compound V is selected from the group consisting of chlorine or bromine.

More particularly, the substituent LG₁ in compound V is bromine.

Particularly, the substituent LG₃ in the compound of formula IV is alkali metal selected from sodium, potassium and lithium.

More particularly, the substituent LG₃ in compound IV is sodium.

The suitable solvent useful is the process step (d) is selected from the group consisting of aliphatic, alicyclic or aromatic hydrocarbons, for example petroleum ether, n-hexane, n-heptane, cyclohexane, methylcyclohexane, benzene, toluene, xylene or decalin; halogenated hydrocarbons, for example chlorobenzene, dichlorobenzene, dichloromethane, chloroform, tetrachloromethane, dichloroethane or trichloroethane; ethers such as diethyl ether, diisopropyl ether, methyl tert-butyl ether, methyl tert-amyl ether, dioxane, tetrahydrofuran, 2-methyl tetrahydrofuran, 1,2-dimethoxyethane, 1,2-diethoxyethane, cyclopentylmethylether or anisole; nitriles such as acetonitrile, propionitrile, n- or isobutyronitrile or benzonitrile; amides such as N,N-dimethylformamide, N,N-dimethylacetamide, N-methylformanilide, N-methylpyrrolidone or hexanlethylphosphoranlide; sulphoxides such as dimethyl sulphoxide; sulphones such as sulpholane; alcohols such as methanol, ethanol, or isopropanol.

The suitable dehydrating reagent that facilitates the reaction depicted in the process step (d) is selected from the group consisting of hydrochloric acid, sulfuric acid, orthoformic acid, phorphorous pentoxide, phosphoryl chloride, calcium oxide, iron (III) chloride, N, N'-dicyclohexylcarbodiimide, cyanuric chloride and burgess reagent.

The process step (d) is carried out within a temperature range of from 15 to 150 °C.

The reaction time is not critical and depends on the batch size, temperature, reagent and solvent employed. Typically, the reaction time may vary from few minutes to several hours.

The process step (d) is usually performed under standard pressure conditions. It is, however, also possible to perform the process step (d) under reduced pressure or elevated pressure conditions.

It is considered to be surprising that the bulky compound such as the compound of formula III can be cyclized with the compound of formula II. The process step (d) is not only novel but also a very innovative and inventive process as a person ordinary skilled in the art would not have anticipated or learnt that the compound of formula III having bulky groups such as T and Q can be cyclized with the compound of formula II. The inventors of the invention unexpectedly and surprisingly were able to cyclize the compound of formula III with the compound of formula II leading to the formation of the compound of formula I in a yield as high as >90%.

**Alternate process steps (i** & ii) (not part of the claimed invention): Alternatively, a compound of formula V can be obtained by halogenating a compound of formula VIII to obtain a compound of formula XI and then reacting the compound of formula XI with a compound of formula VII; 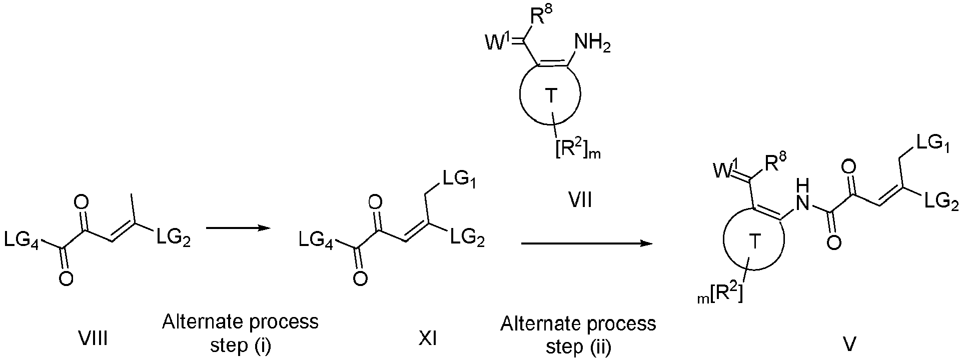 wherein, R², R⁸, m, T, W¹, LG₁, LG₂ and LG₄ are each as defined hereinabove.

In one embodiment the compound of formula XI may be isolated.

In one embodiment the compound of formula XI may not be isolated.

The alternate process step (i) is carried out at a temperature ranging from 0 to 120 °C.

The suitable halogenating agent in the alternate process step (i) is selected from the group consisting of N-chlorosuccinimide, N-bromosuccinimide and N-iodosuccinimide, N-chlorosaccharine, N-bromosaccharine and N-iodosaccharine, N-chlorohydantoine, N-bromohydantoine and N-iodohydantoine, Cl₂, Br₂ or I₂.

The alternate process step (i) can be accelerated by using a radical initiator selected from the group consisting of *hv,* dibenzoyl peroxide, hydrogen peroxide, di(n-propyl)peroxydicarbonate, t-butyl peroxybenzoate, methyl ethyl ketone peroxide, 2,5-dimethyl-2,5-di(t-butylperoxy)-3-hexyne, di(t-butyl)peroxide, acetone peroxide, dicumyl peroxide, azobisisobutyronitrile, bis(2-ethylhexyl)peroxydicarbonate, (peroxybis(propane-2,2-diyl))dibenzene, peracetic acid, metachloroperbenzoic acid, Payne's reagent, magnesium monoperphthalate, trifluroperacetic acid, trichloroperacetic acid, 2, 4-dinitorperbenzoic acid, caro's acid and potassium caroate.

The alternate process step (ii) is carried out at a temperature ranging from 0 to 120 °C.

The alternate process step (ii) can be assisted by a suitable coupling reagent selected from the group consisting of pyridine, 1- or 2- or 3-picoline, triethyl amine, *N,N*-diisopropylethylamine, 2,6-di-tert-butylpyridine, 1,5-diazabicyclo(4.3.0)non-5-ene, 1,8-diazabicycloundec-7-ene, lithium diisopropylamide, sodium bis(trimethylsilyl)amide, and potassium bis(trimethylsilyl)amide or combinations thereof.

**Alternate process steps (iii** & iv) (not part of the claimed invention): Alternatively, the compounds of formula I can be prepared by reacting a compound of formula V with a compound of formula II to obtain a compound of formula XII and then reacting the compound of formula XII with a compound of formula IV; 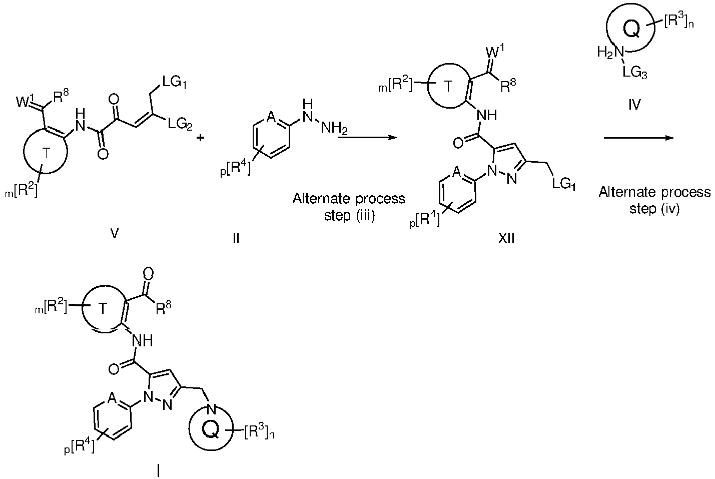 wherein, R², R³, R⁴, R⁸, m, n, p, A, Q, T, W¹ LG₁, LG₂ and LG₃ are each as defined hereinabove.

In one embodiment the compound of formula XII may be isolated.

In one embodiment the compound of formula XII may not be isolated.

The alternate process step (iii) is carried out at a temperature ranging from 15 to 150 °C, optionally using a suitable reagent known in the literature.

The alternate process step (iii) can be assisted by using a suitable dehydrating agent selected from the group consisting of hydrochloric acid, sulfuric acid, orthoformic acid, phorphorous pentoxide, phosphoryl chloride, calcium oxide, iron (III) chloride, N, N'-dicyclohexylcarbodiimide, cyanuric chloride and burgess reagent.

The alternate process step (ii) is carried out at a temperature ranging from 15 to 150 °C.

The suitable solvent in alternate process steps (i), (ii), (iii) and (iv) is selected from the group consisting of petroleum ether, n-hexane, n-heptane, cyclohexane, methylcyclohexane, benzene, toluene, xylene, decalin, chlorobenzene, dichlorobenzene, dichloromethane, chloroform, tetrachloromethane, dichloroethane, trichloroethane, diethyl ether, diisopropyl ether, methyl tert-butyl ether, methyl tert-amyl ether, dioxane, tetrahydrofuran, 2-methyl tetrahydrofuran, 1,2-dimethoxyethane, 1,2-diethoxyethane, cyclopentylmethylether, anisole, acetonitrile, propionitrile, n-butyronitrile, iso-butyronitrile, benzonitrile, N,N-dimethylformamide, N,N-dimethylacetamide, N-methylformanilide, N-methylpyrrolidone, hexan-1-ethylphosphoranlide, dimethyl sulphoxide, sulpholane, methanol, ethanol, isopropanol or combinations thereof.

The compounds, such as, of formulae VII, VI, V, III, and 1 wherein R⁸ is hydrogen, hydroxy, chlorine, bromine, iodine, C₁-C₆-alkoxy, or C₃-C₈-cycloalkoxy is/are converted into the compound of formulae VII, VI, V, III, and 1, respectively, wherein R⁸ is NR^{1a}R^{1b}, wherein, R^{1a} and R^{1b} are each as defined herein above using the processes known in the literature.

For example, a compound of formula VI, wherein R⁸ is OMe, can be converted into a compound of formula VI, wherein R⁸ is OH, by the process reported in CN106565675.

In another example, a compound of formula V, wherein R⁸ is OH, can be converted into a compound of formula V, wherein R⁸ is Cl, by the process reported in Tetrahedron Letters (1993), 34(51), 8267-70.

In another example, a compound of formula XII, wherein R⁸ is OH or Cl, can be converted into a compound of formula XII, wherein R⁸ is NR^{1a}R^{1b}, by the process reported in Journal of Organic Chemistry (2017), 82(19), 10470-10478.

The compounds, such as characterized by formula VI, V, III and XII, wherein W¹ is NR⁵ or S can be converted into the corresponding carbonyl compounds by processes known in the literature.

For example, compounds of formula VI, wherein W¹ is NR⁵, can be converted into compounds of formula VI, wherein W¹ is O, by the process reported in Green Chemistry, Vol.15, Issue 8, pages 2252-2260.

In another example, a compound of formula III, wherein W¹ is S, can be converted into a compound of formula III, wherein W¹ is O, by the processes reported in Journal of Chemical Research, (2003), Synopses, (6), pages 348-350 or Synthesis (2008), (21), 3443-3446, or Chemica Scripta (1994), 24(2), 80-83.

The compounds characterized by formula VI, V, III, XII and VII, wherein W¹ and R⁸ together with the carbon atom to which they are attached form 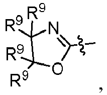 can be converted into the corresponding carboxylic acids by the process reported in Tetrahedron Letters (1986), 27(50), pages 6111-6114 or Advanced Synthesis & Catalysis (2017), 359(15), pages 2596-2604.

For example, compounds of formula XII-a or XII-b can be converted into compounds of formula XII using the process reported in above mentioned literature.

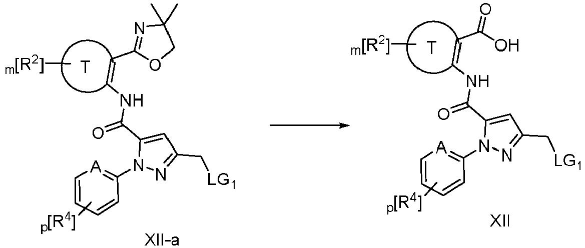

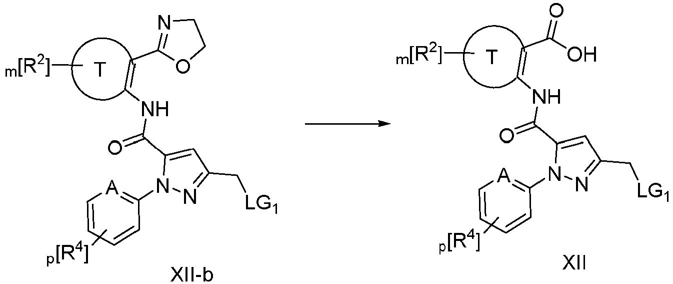

In the preparation of the compound of formula I several novel compounds were being formed.

The novel compound formula XIII, as well as the use of the novel compound of formula VIII as an intermediate in the process of the invention, are also comprised within the scope of the present invention.

Accordingly, the present invention further relates to the use as an intermediate in the process of the invention of a compound of formula VIII 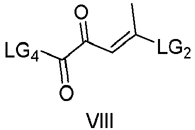 wherein, LG₂ is C₁-C₆-alkoxy or halogen selected from the group consisting of iodide, chloride and bromide; and LG₄ is halogen selected from the group consisting of iodide, chloride and bromide.

The present invention still further relates to a compound of formula XIII, 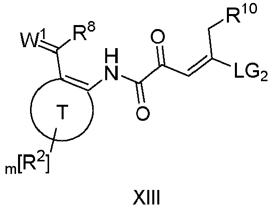 wherein,
R¹⁰ is hydrogen, LG₁, or 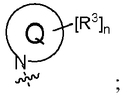
wherein,
   LG₁ is selected from the group consisting of fluorine, chlorine, bromine or iodine,
   R³ is selected from the group consisting of hydrogen, halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl and C₁-C₆-haloalkylsulphonyl,
   Q is a 3-, 4- or 5 membered heterocyclic ring, and
   n is an integer 0 to 4;
R⁸ is selected from the group consisting of hydrogen, hydroxy, chlorine, bromine, iodine, C₁-C₆-alkoxy, C₃-C₈-cycloalkoxy or NR^{1a}R^{1b};
   wherein,
   R^{1a} and R^{1b} are independently selected from the group consisting of hydrogen, C₁-C₆-alkyl, C₁-C₆-haloalkyl, C₃-C₆-cycloalkyl, (C₁-C₆-alkyl)-C₃-C₆-cycloalkyl and (C₃-C₆-cycloalkyl)-C₁-C₆-alkyl, or
   R^{1a} and R^{1b} together with the N atom to which they are attached form N=S(=O)₀₋₂(C₁-C₆-alkyl)₂ or 3- or 4- or 5- or 6- membered heterocyclic ring comprising one or more heteroatoms selected from N, O and S; and the C atom of the heterocyclic ring may be replaced with C(=O), C(=S) and C(=NR⁷),
      wherein,
      the heterocyclic ring may be substituted with at least group selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, C₁-C₆-haloalkylsulphonyl, and
      R⁷ is selected from the group consisting of hydrogen, hydroxy, cyano, C₁-C₆-alkyl, C₁-C₆-haloalkyl, C₁-C₆-alkoxy, C₁-C₆-haloalkoxy, C₃-C₆-cycloalkyl, C₃-C₆-halocycloalkyl, C₃-C₆-cycloalkoxy, C₃-C₆-halocycloalkoxy;
W¹ is selected from O, S or NR⁵;
   R⁵ is selected from the group consisting of hydrogen, hydroxy, cyano, nitro, halogen, C₁-C₆-alkyl, C₁-C₆-haloalkyl, C₁-C₆-alkoxy, C₁-C₆-haloalkoxy, C₃-C₆-cycloalkyl, C₃-C₆-halocycloalkyl;
   or
W¹ and R⁸ together with the carbon atom to which they are attached may form an oxazoline ring 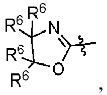 wherein, 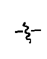
   indicates the point of attachment to T; and
   R⁶ is independently hydrogen or C₁-C₆-alkyl; and
aryl or a heteroaryl ring or ring system;
R² is selected from the group consisting of hydrogen, halogen, cyano, nitro, C₁-C₆-alkyl, C₁-C₆-haloalkyl and C₃-C₆-cycloalkyl;
m is an integer 2; and
   LG₂ is C₁-C₆-alkloxy or halogen selected from the group consisting of iodide, chloride and bromide.

Particularly, the present invention relates to the compound of formula XIII, wherein
R¹⁰ is hydrogen, LG₁, or 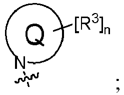 wherein,
   LG₁ is chlorine or bromine,
   R³ is C₁-C₆-haloalkyl,
   Q is a 5 membered heterocyclic ring, and
   n is an integer 1;
R⁸ is selected from the group consisting of hydroxy, chlorine, C₁-C₆-alkoxy, or NR^{1a}R^{1b};
   wherein,
   R^{1a} is hydrogen, and
   R^{1b} is C₁-C₆-alkyl, (C₁-C₆-alkyl)-C₃-C₆-cycloalkyl or (C₃-C₆-cycloalkyl)-C₁-C₆-alkyl;
W¹ is selected from O; T is phenyl; R² is selected from the group consisting of halogen, cyano, C₁-C₆-alkyl; m is an integer 2; and LG₂ is methoxy or ethoxy.

More particularly, the present invention relates to the compound of formula XIII, wherein
wherein,
R¹⁰ is hydrogen, LG₁, or 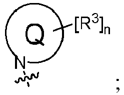 wherein,
   LG₁ is bromine,
   R³ is 5-trifluoro methyl,
   Q is a 2-tetrazolyl ring, and
   n is an integer 1;
R⁸ is NR^{1a}R^{1b};
   wherein,
   R^{1a} is hydrogen, and
   R^{1b} is methyl or 1-cyclopropylethyl;
W¹ is selected from O; T is phenyl; R² is 4-cyano and 2-methyl, or 2-bromo and 4-chloro; m is an integer 2; and LG₂ is methoxy.

Also described herein but not part of the claimed invention are the compounds of formula XI and XII; 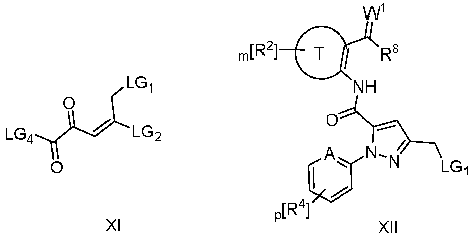 wherein, R², R⁴, R⁸, m, p, A, T, W¹, LG₁, LG₂, LG₄ are each as defined herein above.

The reagents, such as, organic and inorganic acids and organic and inorganic bases known in the art may be used to assist or accelerate or facilitate the process steps (a to d) or alternate process steps (I to iv). Non limiting representative examples of reagents and catalysts useful in the process of the present invention can be seen in the preparation examples.

The present invention is further described in detailed as illustrated in the non-limiting examples. It should be understood that variations and modifications of the processes are possible within the ambit of the invention as defined by the appended claims.

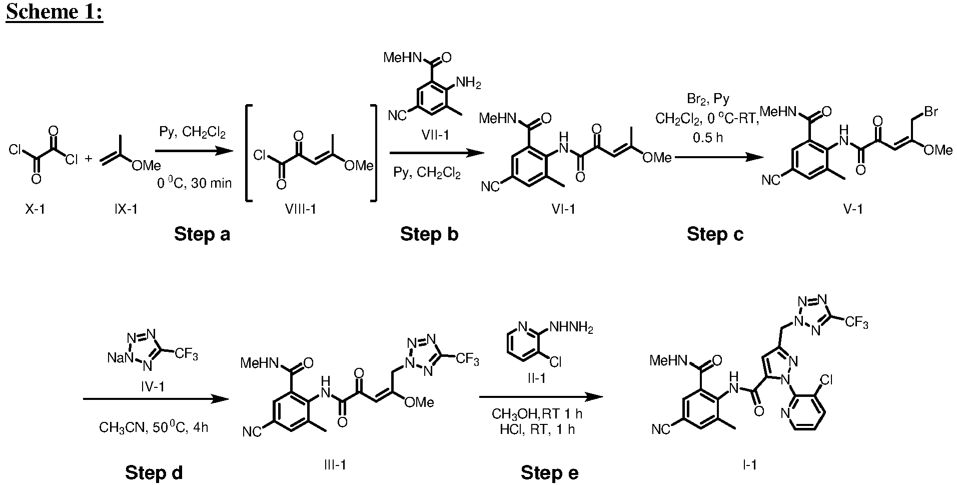

### Step a & b: Synthesis of 5-cyano-2-(4-methoxy-2-oxopent-3-enamido)-N,3-dimethylbenzamide (VI-1):

A solution of oxalyl chloride (X-1) (2.4 mL, 27.7 mmol) in dichloromethane (5 mL) was added to a stirred solution of 2-methoxyprop-1-ene (IX-1) (1.3 mL, 13.87 mmol) and pyridine (2.3 mL, 29.1 mmol) in dichloromethane (15 mL) at 0 °C over 5 min. The resultant mixture was stirred at 0 °C for 30 min and then dichloromethane was distilled off. The residue was dissolved in acetonitrile (20 mL) at 0 °C, and 2-amino-5-cyano-N,3-dimethylbenzamide (VII-1) (2.6 g, 13.87 mmol) was added to the resulting reaction mixture. The reaction mixture was stirred at 50 °C for 2 h. The reaction mixture was cooled and poured onto crushed ice. The precipitate was filtered and washed with water and hexane to obtain a crude product which was purified by flash chromotography to obtain pure 5-cyano-2-(4-methoxy-2-oxopent-3-enamido)-N,3-dimethylbenzamide (VI-1) (1.6 g).

¹H-NMR (400 MHz, CDCl₃) δ 10.54 (bs, 1H), 7.61-7.63 (m, 2H), 6.56 (s, 1H), 6.15-6.16 (m, 1H), 3.83 (s, 3H), 2.98 (d, 3H), 2.45 (s, 3H), 2.31 (s, 3H).

LCMS: [314]^{M-H}.

### Step c: Synthesis of 2-(5-bromo-4-methoxy-2-oxopent-3-enamido)-5-cyano-N,3-dimethylbenzamide (V-1):

A solution of bromine (0.07 mL, 1.3 mmol) in dichloromethane (1 mL) was added drop wise to a stirred solution of 5-cyano-2-(4-methoxy-2-oxopent-3-enamido)-N,3-dimethylbenzamide (VI-1) (0.4 g, 1.3 mmol) in dichloromethane (2 mL) at 0 °C. The resulting reaction mixture was stirred at 0 °C for 15 min followed by addition of a solution of pyridine (0.1 mL, 1.3 mmol) in dichloromethane (0.5 mL) at 0 °C.

After completion of the reaction, the reaction mixture was diluted with dichloromethane (20 mL). The dichloromethane layer was washed subsequently with saturated aqueous sodium bicarbonate solution (20 mL) followed by water (20 mL), then with 2 N aqueous hydrogen chloride (10 mL) and finally with brine solution (20 mL). The dichloromethane layer was dried over anhydrous sodium sulphate and concentrated under reduced pressure to obtain crude 2-(5-bromo-4-methoxy-2-oxopent-3-enamido)-5-cyano-N,3-dimethylbenzamide (V-1; 0.4 g, 1.0 mmol, 78% yield).

¹H-NMR (400 MHz, CDCl₃) δ 10.6 (bs, 1H), 7.61-7.64 (m, 2H), 6.66 (s, 1H), 6.12-6.13 (m, 1H), 4.55 (s, 2H), 3.89 (s, 3H), 2.98 (d, 3H), 2.32 (s, 3H).

LCMS: [392]^{M-H}.

### Step d: Synthesis of 5-cyano-2-(4-methoxy-2-oxo-5-(5-(trifluoromethyl)-2H-tetrazol-2-yl)pent-3-enamido)-N,3-dimethylbenzamide (III-1):

Sodium 5-(trifluoromethyl)tetrazol-1-ide (IV-1) (0.1 g, 0.6 mmol) was added to a stirred solution of 2-(5-bromo-4-methoxy-2-oxopent-3-enamido)-5-cyano-N,3-dimethylbenzamide (V-1) (0.2 g, 0.50 mmol) in acetonitrile (3 mL) at 25 °C. The resulting reaction mixture was heated to 50 °C for 4 h under stirring.

After completion of the reaction, the reaction mixture was cooled to 25 °C and volatiles were removed to obtain a crude 5-cyano-2-(4-methoxy-2-oxo-5-(5-(trifluoromethyl)-2H-tetrazol-2-yl)pent-3-enamido)-N,3-dimethylbenzamide (III-1; 0.2 g, 0.4 mmol, 87% yield).

¹H-NMR (400 MHz, CDCl₃) δ 10.81 (bs, 1H), 7.62-7.65 (m, 2H), 6.84 (s, 1H), 6.44-6.45 (m, 1H), 6.18 (s, 2H), 3.82 (s, 3H), 2.99 (d, 3H), 2.33 (s, 3H).

¹⁹F-NMR (400 MHz, CDCl₃) δ. -63.57 (s)

LCMS: [561]^{M-H}.

### Step e: Synthesis of 1-(3-chloropyridin-2-yl)-N-(4-cyano-2-methyl-6-(methylcarbamoyl)phenyl)-3-((5-(trifluoromethyl)-2H-tetrazol-2-yl)methyl)-1H-pyrazole-5-carboxamide (I-1):

A solution of 3-chloro-2-hydrazinylpyridine (II-1) (32 mg, 0.2 mmol) and 5-cyano-2-(4-methoxy-2-oxo-5-(5-(trifluoromethyl)-2H-tetrazol-2-yl)pent-3-enamido)-N,3-dimethylbenzamide (III-1) (100 mg, 0.2 mmol) in methanol (2 mL) was stirred at 25 °C for 1 h. After completion of the reaction, a solution of 4 M hydrochloric acid in methanol (1 mL) was added to the reaction mixture and allowed to stir at 25 °C for 1 h. Methanol was distilled off under reduced pressure to obtain 1-(3-chloropyridin-2-yl)-N-(4-cyano-2-methyl-6-(methylcarbamoyl)phenyl)-3-((5-(trifluoromethyl)-2H-tetrazol-2-yl)methyl)-1H-pyrazole-5-carboxamide (I-1) (116 mg, 0.2 mmol, 96% yield).

¹H-NMR (400 MHz, DMSO-d6) δ. 10.52 (s, 1H), 8.47 (dd, 1H), 8.36 (m, 1H), 8.15 (dd, 1H), 7.85 (d, 1H), 7.73 (d, 1H), 7.59 (m, 1H), 7.38 (s, 1H), 6.32 (s, 2H), 2.64 (d, 3H), 2.18 (s, 3H),
¹⁹F-NMR (400 MHz, DMSO-d6) δ. -62.61(s)

LCMS: [543]^{M-H}.

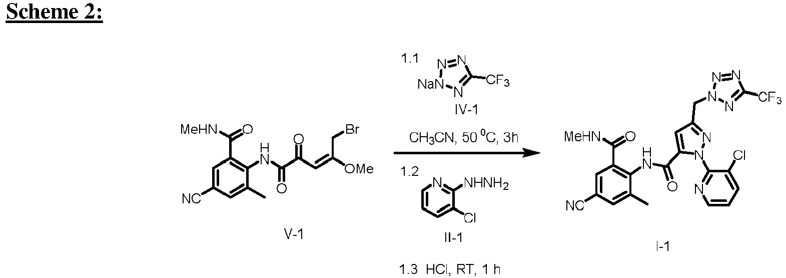

### Synthesis of 1-(3-chloropyridin-2-yl)-N-(4-cyano-2-methyl-6-(methylcarbamoyl)phenyl)-3-((5-(trifluoromethyl)-2H-tetrazol-2-yl)methyl)-1H-pyrazole-5-carboxamide (1-1)

Sodium 5-(trifluoromethyl)tetrazol-1-ide (IV-1) (0.1 g, 0.6 mmol) was added to a stirred solution of 2-(5-bromo-4-methoxy-2-oxopent-3-enamido)-5-cyano-N,3-dimethylbenzamide (V-1) (0.2 g, 0.5 mmol) in acetonitrile (3 mL) at 25 °C. The resulting reaction mixture was heated to 50 °C for 3 h under stirring. After completion of the reaction, the reaction mixture was cooled to 25 °C. 3-chloro-2-hydrazinylpyridine (II-1) (73 mg, 0.5 mmol) was added to the reaction mixture and stirred at 25 °C for 1 h. After completion of the reaction, a solution of 4 M hydrochloric acid in dioxane (1 mL) was added to the reaction mixture and stirred at 25 °C for 1 h. Volatiles were distilled off out under reduced pressure to obtain crude 1-(3-chloropyridin-2-yl)-N-(4-cyano-2-methyl-6-(methylcarbamoyl)phenyl)-3-((5-(trifluoromethyl)-2H-tetrazol-2-yl)methyl)-1H-pyrazole-5-carboxamide (I-1) (201 mg, 0.37 mmol, 74% yield).

¹H-NMR (400 MHz, DMSO-d6) δ. 10.52 (s, 1H), 8.47 (dd, 1H), 8.36 (m, 1H), 8.15 (dd, 1H), 7.85 (d, 1H), 7.73 (d, 1H), 7.59 (m, 1H), 7.38 (s, 1H), 6.32 (s, 2H), 2.64 (d, 3H), 2.18 (s, 3H), ¹⁹F-NMR (400 MHz, DMSO-d6) δ. -62.61(s)
LCMS: [543]^{M-H}

## Claims

1. A process for preparing a compound of formula I, 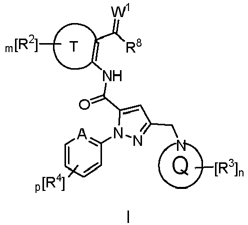 wherein,
R⁸ is selected from the group consisting of hydrogen, hydroxy, chlorine, bromine, iodine, C₁-C₆-alkoxy, C₃-C₈-cycloalkoxy or NR^{1a}R^{1b};
wherein,
R^{1a} and R^{1b} are independently selected from the group consisting of hydrogen, C₁-C₆-alkyl, C₁-C₆-haloalkyl, C₃-C₆-cycloalkyl, (C₁-C₆-alkyl)-C₃-C₆-cycloalkyl and (C₃-C₆-cycloalkyl)-C₁-C₆-alkyl, or
R^{1a} and R^{1b} together with the N atom to which they are attached form N=S(=O)₀₋₂(C₁-C₆-alkyl)₂ or 3- or 4- or 5- or 6- membered heterocyclic ring comprising one or more heteroatoms selected from N, O and S; and the C atom of the heterocyclic ring may be replaced with C(=O), C(=S) and C(=NR⁷), wherein,
the heterocyclic ring may be substituted with one or more substituent selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, C₁-C₆-haloalkylsulphonyl, and
R⁷ is selected from the group consisting of hydrogen, hydroxy, cyano, C₁-C₆-alkyl, C₁-C₆-haloalkyl, C₁-C₆-alkoxy, C₁-C₆-haloalkoxy, C₃-C₆-cycloalkyl, C₃-C₆-halocycloalkyl, C₃-C₆-cycloalkoxy, C₃-C₆-halocycloalkoxy;
W¹ is selected from O, S or NR⁵,
wherein, R⁵ is selected from the group consisting of hydrogen, hydroxy, cyano, nitro, halogen, C₁-C₆-alkyl, C₁-C₆-haloalkyl, C₁-C₆-alkoxy, C₁-C₆-haloalkoxy, C₃-C₆-cycloalkyl, C₃-C₆-halocycloalkyl;
or
W¹ and R⁸ together with the carbon atom to which they are attached may form an oxazoline ring 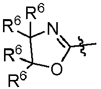 ,
wherein, 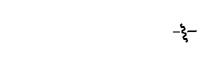
indicates the point of attachment to T, and
R⁶ is independently hydrogen or C₁-C₆-alkyl;
aryl or a heteroaryl ring or ring system;
R² is selected from the group consisting of hydrogen, halogen, cyano, nitro, C₁-C₆-alkyl, C₁-C₆-haloalkyl and C₃-C₆-cycloalkyl;
A is N or C;
R³ is selected from the group consisting of hydrogen, halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl and C₁-C₆-haloalkylsulphonyl;
R⁴ is selected from the group consisting of hydrogen, halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, C₁-C₆-haloalkylsulphonyl or NR^{a}R^{b};
wherein, R^{a} and R^{b} are independently selected from the group consisting of hydrogen, C₁-C₆-alkyl, C₃-C₆-cycloalkyl or R^{a} and R^{b} together with the N atom to which they are attached form a substituted or unsubstituted 3- to 6- membered heterocylic ring,
wherein, the substitution on 3- to 6- membered heterocyclic ring is selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl and C₁-C₆-haloalkylsulphonyl;
Q is a 3-, 4- or 5 membered heterocyclic ring;
n is an integer 0 to 4;
m is an integer 0 to 6; and
p is an integer 0 to 5;
said process comprising the steps of:
a) obtaining a compound of formula VI by reacting a compound of formula VIII and a compound of formula VII in a suitable solvent optionally using a suitable coupling reagent; 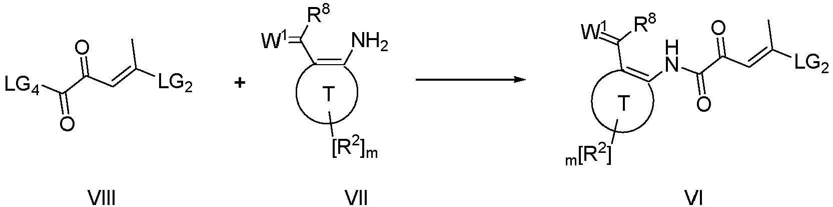 wherein,
LG₂ is selected from the group consisting of fluorine, chlorine, bromine, iodine or OR¹², wherein, R¹² is selected from the group consisting of hydrogen, substituted or unsubstituted C₁-C₆-alkyl, substituted or unsubstituted aryl-C₁-C₆-alkyl, substituted or unsubstituted aryl, -(C=O)-C₁-C₆-alkyl, -(C=O)-C₁-C₆-haloalkyl, -(C=O)O-C₁-C₆-alkyl, -(C=O)O-haloC₁-C₆-alkyl, SO₂-C₁-C₆-alkyl, SO₂-C₁-C₆-haloalkyl, substituted or unsubstituted SOz-aryl and alkylthio;
LG₄ is selected from the group consisting of chlorine, bromine, iodine, N(OR¹³)R¹³, OR¹³ or 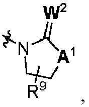 wherein, R¹³ is selected from the group consisting of hydrogen, substituted or unsubstituted C₁-C₆-alkyl, substituted or unsubstituted aryl-C₁-C₆-alkyl, substituted or unsubstituted aryl, -(C=O)-C₁-C₆-alkyl, -(C=O)-C₁-C₆-haloalkyl, -(C=O)O-C₁-C₆-alkyl, -(C=O)O-haloC₁-C₆-alkyl, SO₂-C₁-C₆-alkyl, SOz-Ci-Ce-haloalkyl, substituted or unsubstituted SOz-aryl and alkylthio, wherein, the substitutions on C₁-C₆-alkyl, aryl-C₁-C₆-alkyl, aryl and SO₂-aryl of R¹² and R¹³ are independently selected from the group consisting of halogen, cyano, nitro, hydroxy, C₁-C₆-alkyl, C₁-C₆-haloalkyl, C₃-C₆-cycloalkyl, C₃-C₆-halocycloalkyl, Ci-Ce-alkoxy, C₁-C₆-haloalkoxy, Ci-Ce-alkylthio, C₁-C₆-alkylsulphinyl, C₁-C₆-alkylsulphonyl, C₁-C₆-haloalkylthio, C₁-C₆-haloalkylsulphinyl and C₁-C₆-haloalkylsulphonyl,
W² and A¹ are independently selected from O, S or NR⁵, and
R⁹ is selected from the group consisting of hydrogen, halogen, cyano, C₁-C₆-alkyl, C₁-C₆-haloalkyl, C₁-C₆-alkoxy, C₁-C₆-haloalkoxy, C₃-C₆-cycloalkyl, C₃-C₆-halocycloalkyl, C₁-C₆-alkylthio, C₁-C₆-haloalkylthio, C₁-C₆-alkylsulphinyl, C₁-C₆-haloalkylsulphinyl, C₁-C₆-alkylsulphonyl, C₁-C₆-haloalkylsulphonyl, aryl and arylalkyl;
R², R⁵, R⁸, m, T, and W¹ are each as defined herein above;
b) halogenating the compound of formula VI using a suitable halogenating agent in a suitable solvent, optionally in the presence of a radical initiator to obtain a compound of formula V; 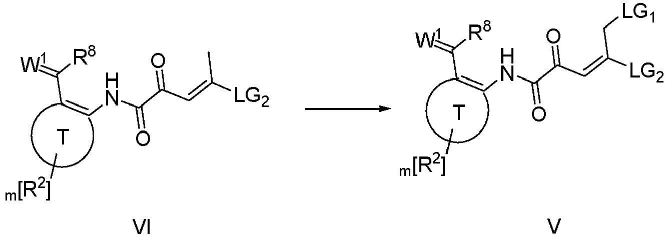 wherein,
LG₁ is selected from the group consisting of fluorine, chlorine, bromine or iodine; and
R², R⁸, m, T, W¹ and LG₂ are each as defined herein above,
c) reacting the compound of formula V and a compound of formula IV in a suitable solvent to obtain a compound of formula III; 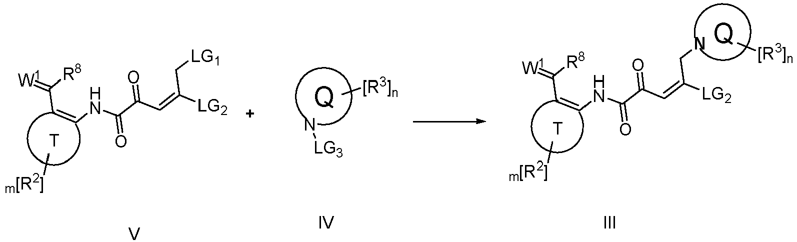 wherein,
LG₃ is selected from the group consisting of hydrogen, halogen, Si(R¹¹)₃ and alkali metal selected from sodium, potassium and lithium,
wherein, R¹¹ is selected from the group consisting of hydrogen, halogen, hydroxy, C₁-C₆-alkyl, C₁-C₆-haloalkyl, C₃-C₆-cycloalkyl, C₃-C₆-halocycloalkyl, C₁-C₆-alkoxy and C₁-C₆-haloalkoxy;
R², R³, R⁸, m, n, Q, T, W¹, LG₁ and LG₂ are each as defined herein above;
d) the compound of formula III is reacted with a compound of formula II in a suitable solvent and a suitable dehydrating agent to obtain the compound of formula I; 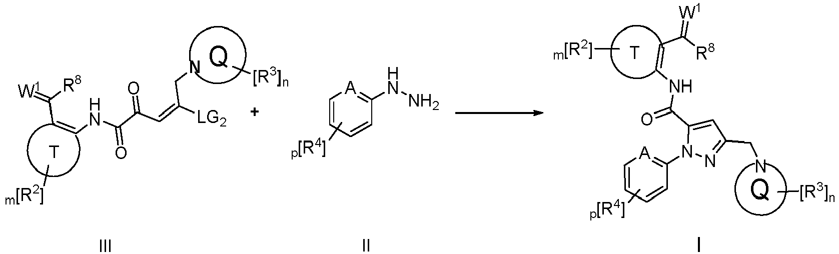 wherein,
the compounds of formulae VI, V and III may or may not be isolated,
R², R³, R⁴, R⁸, m, n, p, A, Q, T, W¹ and LG₂ are each as defined herein above, and
optionally, any or all of the compounds of formulae VII, VI, V, and III, wherein R⁸ is hydrogen, hydroxy, chlorine, bromine, iodine, C₁-C₆-alkoxy, or C₃-C₈-cycloalkoxy is/are converted into the compound of formulae VII, VI, V, and III, respectively, wherein R⁸ is NR^{1a}R^{1b}, and R^{1a} and R^{1b} are each as defined herein above.

2. The process as claimed in claim 1, wherein,
R⁸ is selected from the group consisting of hydroxy, chlorine, C₁-C₆-alkoxy or NR^{1a}R^{1b},
wherein, R^{1a} and R^{1b} are independently selected from the group consisting of hydrogen, C₁-C₆-alkyl, (C₁-C₆-alkyl)-C₃-C₆-cycloalkyl and (C₃-C₆-cycloalkyl)-C₁-C₆-alkyl;
W¹ is O; T is phenyl; R² is selected from the group consisting of hydrogen, halogen, cyano and C₁-C₆-alkyl; A is N; R³ is selected from the group consisting of hydrogen, halogen and C₁-C₆-haloalkyl; R⁴ is selected from the group consisting of hydrogen or halogen; Q is a 5 membered heterocyclic ring; n is an integer 1; m is an integer 2; p is an integer 1 or 2;
LG₂ is selected from the group consisting of chlorine, bromine or OR¹²,
wherein, R¹² is C₁-C₆-alkyl;
LG₄ is selected from the group consisting of chlorine or OR¹³,
wherein, R¹³ is selected from the group consisting of hydrogen or C₁-C₆-alkyl; and
LG₁ is selected from the group consisting of chlorine or bromine; and LG₃ is alkali metal selected from sodium, potassium and lithium.

3. The process as claimed in claim 1,
wherein,
R⁸ is NR^{1a}R^{1b}, wherein, R^{1a} is hydrogen; and R^{1b} is C₁-C₆-alkyl, (C₁-C₆-alkyl)-C₃-C₆-cycloalkyl and (C₃-C₆-cycloalkyl)-C₁-C₆-alkyl; W¹ is O; T is phenyl; R² is selected from the group consisting of halogen, cyano and C₁-C₆-alkyl; A is N; R³ is C₁-C₆-haloalkyl; R⁴ is halogen; Q is a tetrazolyl ring; n is an integer 1; m is an integer 2; p is an integer 1; LG₂ is OR¹², wherein, R¹² is C₁-C₃-alkyl; LG₄ is chlorine; LG₁ is bromine; and LG₃ is sodium.

4. The process as claimed in claim 1,
wherein,
R⁸ is NR^{1a}R^{1b}, wherein, R^{1a} is hydrogen; and R^{1b} is methyl or 1-cyclopropylethyl; W¹ is O; T is phenyl; R² is 4-cyano and 2-methyl, or 2-bromo and 4-chloro; A is N; R³ is 5-trifluoro methyl; R⁴ is 3-chlorine; Q is a 2-tetrazolyl ring; n is an integer 1; m is an integer 2; p is an integer 1; LG₂ is methoxy; LG₄ is chlorine; LG₁ is bromine; and LG₃ is sodium.

5. The process as claimed in claim 1, wherein the said process comprises a pre-step of obtaining the compound of formula VIII by reacting a compound of formula IX and a compound of formula X in a suitable solvent and optionally a suitable reagent, 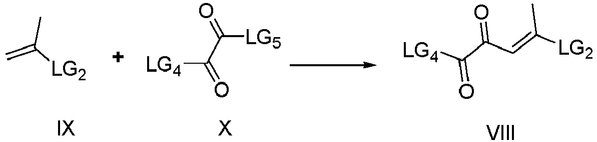 wherein,
LG₅ is hydroxy or halogen; and LG₂ and LG₄ are each as defined in claim 1;
the suitable solvent is selected from the group consisting of petroleum ether, n-hexane, n-heptane, cyclohexane, methylcyclohexane, benzene, toluene, xylene, decalin, chlorobenzene, dichlorobenzene, dichloromethane, chloroform, tetrachloromethane, dichloroethane, trichloroethane, diethyl ether, diisopropyl ether, methyl tert-butyl ether, methyl tert-amyl ether, dioxane, tetrahydrofuran, 2-methyl tetrahydrofuran, 1,2-dimethoxyethane, 1,2-diethoxyethane, cyclopentylmethylether, anisole, acetonitrile, propionitrile, n-butyronitrile, iso-butyronitrile, benzonitrile or combinations thereof;
the suitable reagent is selected from the group consisting of pyridine, 1- or 2- or 3-picoline, triethyl amine, N,N-diisopropylethylamine, 2,6-di-tert-butylpyridine, 1,5-diazabicyclo(4.3.0)non-5-ene, 1,8-diazabicycloundec-7-ene, lithium diisopropylamide, sodium bis(trimethylsilyl)amide, and potassium bis(trimethylsilyl)amide or combinations thereof; and
the compound of formula VIII may or may not be isolated.

6. The process as claimed in claim 1, wherein the suitable solvent in steps (a), (b), (c) and (d) is selected from the group consisting of aliphatic, alicyclic or aromatic hydrocarbons, halogenated hydrocarbons, ethers, nitriles, amides, alcohols or combinations thereof.

7. The process as claimed in claim 1, wherein the suitable solvent in steps (a), (b), (c) and (d) is selected from the group consisting of petroleum ether, n-hexane, n-heptane, cyclohexane, methylcyclohexane, benzene, toluene, xylene, decalin, chlorobenzene, dichlorobenzene, dichloromethane, chloroform, tetrachloromethane, dichloroethane, trichloroethane, diethyl ether, diisopropyl ether, methyl tert-butyl ether, methyl tert-amyl ether, dioxane, tetrahydrofuran, 2-methyl tetrahydrofuran, 1,2-dimethoxyethane, 1,2-diethoxyethane, cyclopentylmethylether, anisole, acetonitrile, propionitrile, n-butyronitrile, iso-butyronitrile, benzonitrile, N,N-dimethylformamide, N,N-dimethylacetamide, N-methylformanilide, N-methylpyrrolidone, hexan-1-ethylphosphoranlide, dimethyl sulphoxide, sulpholane, methanol, ethanol, isopropanol or combinations thereof.

8. The process as claimed in claim 1, wherein
a) the step (a) is carried out at a temperature ranging from 0 to 120 °C; and
b) the suitable coupling reagent in step (a) is selected from the group consisting of pyridine, 1- or 2- or 3-picoline, triethyl amine, *N,N*-diisopropylethylamine, 2,6-di-tert-butylpyridine, 1,5-diazabicyclo(4.3.0)non-5-ene, 1,8-diazabicycloundec-7-ene, lithium diisopropylamide, sodium bis(trimethylsilyl)amide, and potassium bis(trimethylsilyl)amide or combinations thereof.

9. The process as claimed in claim 1, wherein
a) the step (b) is carried out at a temperature ranging from 0 to 120 °C; and
b) the suitable halogenating agent in step (b) is selected from the group consisting of N-chlorosuccinimide, N-bromosuccinimide and N-iodosuccinimide, N-chlorosaccharine, N-bromosaccharine and N-iodosaccharine, N-chlorohydantoine, N-bromohydantoine and N-iodohydantoine, Cl₂, Br₂ or I₂; and
c) the radical initiator in step (b) is selected from the group consisting of *hv*, dibenzoyl peroxide, hydrogen peroxide, di(n-propyl)peroxydicarbonate, t-butyl peroxybenzoate, methyl ethyl ketone peroxide, 2,5-dimethyl-2,5-di(t-butylperoxy)-3-hexyne, di(t-butyl)peroxide, acetone peroxide, dicumyl peroxide, azobisisobutyronitrile, bis(2-ethylhexyl)peroxydicarbonate, (peroxybis(propane-2,2-diyl))dibenzene, peracetic acid, metachloroperbenzoic acid, Payne's reagent, magnesium monoperphthalate, trifluroperacetic acid, trichloroperacetic acid, 2, 4-dinitorperbenzoic acid, caro's acid and potassium caroate.

10. The process as claimed in claim 1, wherein the step (c) is carried out at a temperature ranging from 15 to 150 °C.

11. The process as claimed in claim 1, wherein
a) the step (d) is carried out at a temperature ranging from 15 to 150 °C; and
b) the suitable dehydrating agent in step (d) is selected from the group consisting of hydrochloric acid, sulfuric acid, orthoformic acid, phorphorous pentoxide, phosphoryl chloride, calcium oxide, iron (III) chloride, N, N'-dicyclohexylcarbodiimide, cyanuric chloride and burgess reagent.

12. Use of a compound of formula VIII, 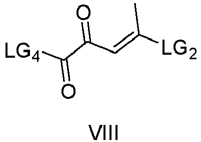 wherein, LG₂ is C₁-C₆-alkoxy or halogen selected from the group consisting of iodide, chloride and bromide; and LG₄ is halogen selected from the group consisting of iodide, chloride and bromide, as an intermediate in the process for the synthesis of a compound of formula I as claimed in claim 1.

13. A compound of formula XIII, 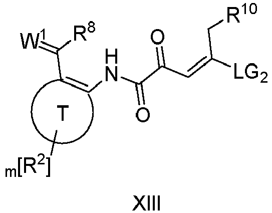 wherein,
R¹⁰ is hydrogen, LG₁, or 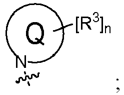
wherein,
LG₁ is selected from the group consisting of fluorine, chlorine, bromine or iodine,
R³ is selected from the group consisting of hydrogen, halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, Ci-Ce-alkylsulphinyl, C₁-C₆-alkylsulphonyl, Ci-Ce-haloalkylthio, C₁-C₆-haloalkylsulphinyl and C₁-C₆-haloalkylsulphonyl,
Q is a 3-, 4- or 5 membered heterocyclic ring, and
n is an integer 0 to 4;
R⁸ is selected from the group consisting of hydrogen, hydroxy, chlorine, bromine, iodine, C₁-C₆-alkoxy, C₃-C₈-cycloalkoxy or NR^{1a}R^{1b};
wherein,
R^{1a} and R^{1b} are independently selected from the group consisting of hydrogen, C₁-C₆-alkyl, C₁-C₆-haloalkyl, C₃-C₆-cycloalkyl, (C₁-C₆-alkyl)-C₃-C₆-cycloalkyl and (C₃-C₆-cycloalkyl)-C₁-C₆-alkyl, or
R^{1a} and R^{1b} together with the N atom to which they are attached form N=S(=O)₀₋₂(C₁-C₆-alkyl)₂ or 3- or 4- or 5- or 6- membered heterocyclic ring comprising one or more heteroatoms selected from N, O and S; and the C atom of the heterocyclic ring may be replaced with C(=O), C(=S) and C(=NR⁷),
wherein,
the heterocyclic ring may be substituted with at least group selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, C₁-C₆-haloalkylsulphonyl, and
R⁷ is selected from the group consisting of hydrogen, hydroxy, cyano, C₁-C₆-alkyl, C₁-C₆-haloalkyl, C₁-C₆-alkoxy, C₁-C₆-haloalkoxy, C₃-C₆-cycloalkyl, C₃-C₆-halocycloalkyl, C₃-C₆-cycloalkoxy, C₃-C₆-halocycloalkoxy;
W¹ is selected from O, S or NR⁵;
R⁵ is selected from the group consisting of hydrogen, hydroxy, cyano, nitro, halogen, C₁-C₆-alkyl, C₁-C₆-haloalkyl, C₁-C₆-alkoxy, C₁-C₆-haloalkoxy, C₃-C₆-cycloalkyl, C₃-C₆-halocycloalkyl;
or
W¹ and R⁸ together with the carbon atom to which they are attached may form an oxazoline ring 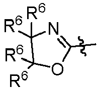 wherein, 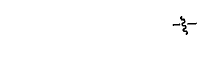
indicates the point of attachment to T; and
R⁶ is independently hydrogen or C₁-C₆-alkyl; and
aryl or a heteroaryl ring or ring system;
R² is selected from the group consisting of hydrogen, halogen, cyano, nitro, C₁-C₆-alkyl, C₁-C₆-haloalkyl and C₃-C₆-cycloalkyl;
m is an integer 2; and
LG₂ is C₁-C₆-alkloay or halogen selected from the group consisting of iodide, chloride and bromide.

14. The compound as claimed in claim 13, wherein,
R¹⁰ is hydrogen, LG₁, or 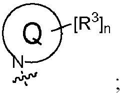 wherein,
LG₁ is chlorine or bromine,
R³ is C₁-C₆-haloalkyl,
Q is a 5 membered heterocyclic ring, and
n is an integer 1;
R⁸ is selected from the group consisting of hydroxy, chlorine, C₁-C₆-alkoxy, or NR^{1a}R^{1b};
wherein,
R^{1a} is hydrogen, and
R^{1b} is C₁-C₆-alkyl, (C₁-C₆-alkyl)-C₃-C₆-cycloalkyl or (C₃-C₆-cycloalkyl)-C₁-C₆-alkyl;
W¹ is selected from O; T is phenyl; R² is selected from the group consisting of halogen, cyano, C₁-C₆-alkyl; m is an integer 2; and LG₂ is methoxy or ethoxy.

15. The compound as claimed in claim 13,
wherein,
R¹⁰ is hydrogen, LG₁, or 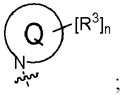 wherein,
LG₁ is bromine,
R³ is 5-trifluoro methyl,
Q is a 2-tetrazolyl ring, and
n is an integer 1;
R⁸ is NR^{1a}R^{1b};
wherein,
R^{1a} is hydrogen, and
R^{1b} is methyl or 1-cyclopropylethyl;
W¹ is selected from O; T is phenyl; R² is 4-cyano and 2-methyl, or 2-bromo and 4-chloro; m is an integer 2; and LG₂ is methoxy.

## Patentansprüche

1. Prozess zur Herstellung einer Verbindung der Formel I, 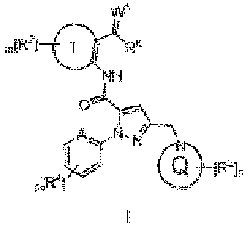 wobei
R⁸ ausgewählt ist aus der Gruppe, bestehend aus Wasserstoff, Hydroxy, Chlor, Brom, Jod, C₁-C₆-Alkoxy, C₃-C₈-Cycloalkoxy oder NR^{1a}R^{1b};
wobei
R^{1a} und R^{1b} unabhängig voneinander ausgewählt sind aus der Gruppe, bestehend aus Wasserstoff, C₁-C₆-Alkyl, C₁-C₆-Halogenalkyl, C₃-G₆-Cycloalkyl, (C₁-C₆-Alkyl)-C₃-C₆-Cycloalkyl und (C₃-C₆-Cycloalkyl)-C₁-C₆-Alkyl oder
R^{1a} und R^{1b} zusammen mit dem N-Atom, an das sie gebunden sind, N=S(=O)₀₋₂(C₁-C₆-Alkyl)₂ oder einen 3- oder 4- oder 5- oder 6-gliedrigen heterocyclischen Ring bilden, der ein oder mehrere Heteroatome, ausgewählt aus N, O und S, umfasst; und das C-Atom des heterocyclischen Rings durch C(=O), C(=S) und C(=NR⁷) ersetzt sein kann,
wobei
der heterocyclische Ring mit einem oder mehreren Substituenten substituiert sein kann, die ausgewählt sind aus der Gruppe, bestehend aus Halogen, Cyano, Nitro, Hydroxy, C₁-G₆-Alkyl, C₁-C₆-Haloalkyl, C₃-C₆-Cycloalkyl, C₃-C₆-Halocycloalkyl, C₁-C₆-Alkoxy, C₁-C₆-Haloalkoxy, C₁-C₆-Alkylthio, C₁-C₆-Alkylsulphinyl, C₁-C₆-Alkylsulphonyl, C₁-C₆-Haloalkylthio, C₁-C₆-Haloalkylsulphinyl, C₁-C₆-Haloalkylsulphonyl und
R⁷ ausgewählt ist aus der Gruppe, bestehend aus Wasserstoff, Hydroxy, Cyano, C₁-C₆-Alkyl, C₁-C₆-Halogenalkyl, C₁-C₆-Alkoxy, C₁-C₆-Haloalkoxy, C₃-C₆-Cycloalkyl, C₃-C₆-Halocycloalkyl, C₃-C₆-Cycloalkoxy, C₃-C₆-Halocycloalkoxy;
W¹ ausgewählt ist aus O, S oder NR⁵,
wobei R⁵ ausgewählt ist aus der Gruppe, bestehend aus Wasserstoff, Hydroxy, Cyano, Nitro, Halogen, C₁-C₆-Alkyl, C₁-C₆-Haloalkyl, C₁-C₆-Alkoxy, C₁-C₆-Haloalkoxy, C₃-C₆-Cycloalkyl, C₃-C₆-Halocycloalkyl;
oder
W¹ und R⁸ zusammen mit dem Kohlenstoffatom, an das sie gebunden sind, einen Oxazolinring 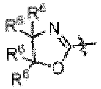 bilden können,
wobei 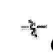
den Befestigungspunkt von T anzeigt, und
R⁶ unabhängig Wasserstoff oder C₁-C₆-Alkyl ist;
Aryl- oder Heteroarylring oder -ringsystem;
R² ausgewählt ist aus der Gruppe, bestehend aus Wasserstoff, Halogen, Cyano, Nitro, C₁-C₆-Alkyl, C₁-C₆-Halogenalkyl und C₃-C₆-Cycloalkyl;
A N oder C ist;
R³ ausgewählt ist aus der Gruppe, bestehend aus Wasserstoff, Halogen, Cyano, Nitro, Hydroxy, C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Haloalkoxy, C₁-C₆-Alkylthio, C₁-C₆-Alkylsulphinyl, C₁-C₆-Alkylsulphonyl, C₁-C₆-Haloalkylthio, C₁-C₆-Haloalkylsulfinyl und C₁-C₆-Haloalkylsulphonyl;
R⁴ ausgewählt ist aus der Gruppe, bestehend aus Wasserstoff, Halogen, Cyano, Nitro, Hydroxy, C₁-C₆-Alkyl, C₁-C₆-Haloalkyl, C₃-C₆-Cycloalkyl, C₃-C₆-Halocycloalkyl, C₁-C₆-Alkoxy, C₁-C₆-Haloalkoxy, C₁-C₆-Alkylthio, C₁-C₆-Alkylsulphinyl, C₁-C₆-Alkylsulphonyl, C₁-C₆-Haloalkylthio, C₁-C₆-Haloalkylsulphinyl, C₁-C₆-Haloalkylsulphonyl oder NR^{a}R^{b};
wobei R^{a} und R^{b} unabhängig voneinander ausgewählt sind aus der Gruppe, bestehend aus Wasserstoff, C₁-C₆-Alkyl, C₃-C₆-Cycloalkyl oder R^{a} und R^{b} zusammen mit dem N-Atom, an das sie gebunden sind, einen substituierten oder unsubstituierten 3- bis 6-gliedrigen heterocyclischen Ring bilden,
wobei die Substitution am 3- bis 6-gliedrigen heterocyclischen Ring ausgewählt ist aus der Gruppe, bestehend aus Halogen, Cyano, Nitro, Hydroxy, C₁-C₆-Alkyl, C₁-C₆-Haloalkyl, C₃-C₆-Cycloalkyl, C₃-C₆-Halocycloalkyl, C₁-C₆-Alkoxy, C₁-C₆-Haloalkoxy, C₁-C₆-Alkylthio, C₁-C₆-Alkylsulphinyl, C₁-C₆-Alkylsulphonyl, C₁-C₆-Haloalkylthio, C₁-C₆-Haloalkylsulphinyl und C₁-C₆-Haloalkylsulphonyl;
Q ein 3-, 4- oder 5-gliedriger heterocyclischer Ring ist;
n eine ganze Zahl von 0 bis 4 ist;
m eine ganze Zahl von 0 bis 6 ist; und
p eine ganze Zahl von 0 bis 5 ist;
der Prozess umfassend die folgenden Schritte:
a) Erhalten einer Verbindung der Formel VI durch Umsetzen einer Verbindung der Formel VIII und einer Verbindung der Formel VII in einem geeigneten Lösungsmittel, gegebenenfalls unter Verwendung eines geeigneten Kupplungsreagens; 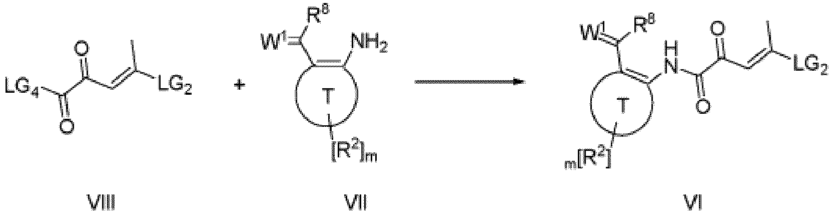
wobei
LG₂ ausgewählt ist aus der Gruppe, bestehend aus Fluor, Chlor, Brom, Jod oder OR¹²,
wobei R¹² ausgewählt ist aus der Gruppe, bestehend aus Wasserstoff, substituiertem oder unsubstituiertem C₁-C₆-Alkyl, substituiertem oder unsubstituiertem Aryl-C₁-C₆-Alkyl, substituiertem oder unsubstituiertem Aryl, - (C=O)-C₁-C₆-Alkyl, -(C=O)-C₁-C₆-Haloalkyl, -(C=O)O-C₁-C₆-Alkyl, -(C=O)O-haloC₁-C₆-Alkyl, SO₂-C₁-C₆-Alkyl, SO₂-C₁-C₆-Halogenalkyl, substituiertem oder unsubstituiertem SO₂-Aryl und Alkylthio;
LG₄ ausgewählt ist aus der Gruppe, bestehend aus Chlor, Brom, Jod, N(OR¹³)R¹³, OR¹³ oder 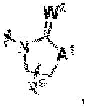
wobei R¹³ ausgewählt ist aus der Gruppe, bestehend aus Wasserstoff, substituiertem oder unsubstituiertem C₁-C₆-Alkyl, substituiertem oder unsubstituiertem Aryl-C₁-C₆-Alkyl, substituiertem oder unsubstituiertem Aryl, - (C=O)-C₁-C₆-Alkyl, -(C=O)-C₁-C₆-Haloalkyl, -(C=O)O-C₁-C₆-Alkyl, -(C=O)O-haloC₁-C₆-Alkyl, SO₂-C₁-C₆-Alkyl, SO₂-C₁-C₆-Halogenalkyl, substituiertem oder unsubstituiertem SO₂-Aryl und Alkylthio,
wobei die Substitutionen an C₁-C₆-Alkyl, Aryl-C₁-C₆-Alkyl, Aryl und SO₂-Aryl von R¹² und R¹³ unabhängig voneinander ausgewählt sind aus der Gruppe, bestehend aus Halogen, Cyano, Nitro, Hydroxy, C₁-C₆-Alkyl, C₁-C₆-Haloalkyl, C₃-C₆-Cycloalkyl, C₃-C₆-Halocycloalkyl, C₁-C₆-Alkoxy, C₁-C₆-Haloalkoxy, C₁-C₆-Alkylthio, C₁-C₆-Alkylsulphinyl, C₁-C₆-Alkylsulphonyl, C₁-C₆-Haloalkylthio, C₁-C₆-Haloalkylsulphinyl und C₁-C₆-Haloalkylsulphonyl,
W² und A¹ unabhängig voneinander ausgewählt sind aus O, S oder NR⁵ und R⁹ ausgewählt ist aus der Gruppe, bestehend aus Wasserstoff, Halogen, Cyano, C₁-C₆-Alkyl, C₁-C₆-Halogenalkyl, C₁-C₆-Alkoxy, C₁-C₆-Haloalkoxy, C₃-C₆-Cycloalkyl, C₃-C₆-Halocycloalkyl, C₁-C₆-Alkylthio, C₁-C₆-Haloalkylthio, C₁-C₆-Alkylsulphinyl, C₁-C₆-Haloalkylsulfinyl, C₁-C₆-Alkylsulfonyl, C₁-C₆-Haloalkylsulfonyl, Aryl und Arylalkyl;
R², R⁵, R⁸, m, T und W¹ jeweils wie hier oben definiert sind;
b) Halogenieren der Verbindung der Formel VI mit einem geeigneten halogenierenden Mittel in einem geeigneten Lösungsmittel, gegebenenfalls in Gegenwart eines Radikalinitiators, um eine Verbindung der Formel V zu erhalten; 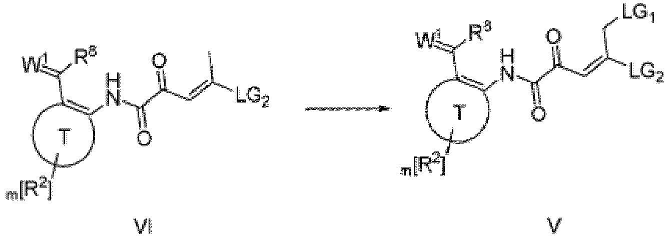
wobei
LG₁ ausgewählt ist aus der Gruppe, bestehend aus Fluor, Chlor, Brom oder Jod; und
R², R⁸, m, T, W¹ und LG₂ jeweils wie oben definiert sind,
c) Umsetzen der Verbindung der Formel V und einer Verbindung der Formel IV in einem geeigneten Lösungsmittel, um eine Verbindung der Formel III zu erhalten; 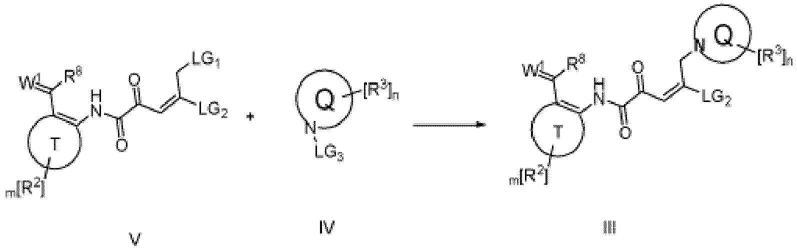
wobei
LG₃ ausgewählt ist aus der Gruppe, bestehend aus Wasserstoff, Halogen, Si(R¹¹)₃ und Alkalimetall, ausgewählt aus Natrium, Kalium und Lithium,
wobei R¹¹ ausgewählt ist aus der Gruppe, bestehend aus Wasserstoff, Halogen, Hydroxy, C₁-C₆-Alkyl, C₁-C₆-Halogenalkyl, C₃-C₆-Cycloalkyl, C₃-C₆-Halocycloalkyl, C₁-C₆-Alkoxy und C₁-C₆-Haloalkoxy;
R², R³, R⁸, m, n, Q, T, W¹, LG₁ und LG₂ jeweils wie oben definiert sind;
d) Umsetzen der Verbindung der Formel III mit einer Verbindung der Formel II in einem geeigneten Lösungsmittel und einem geeigneten dehydratisierenden Mittel, um die Verbindung der Formel I zu erhalten; 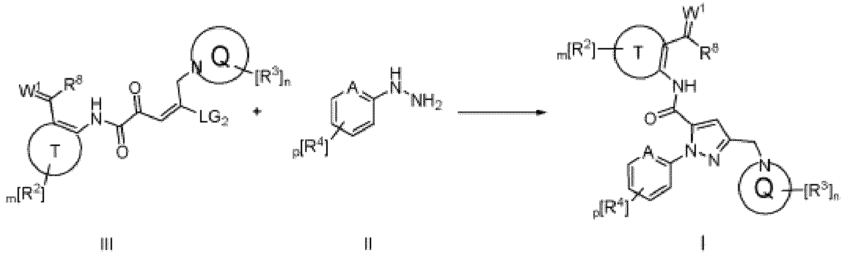
wobei
die Verbindungen der Formeln VI, V und III isoliert sein können oder nicht,
R², R³, R⁴, R⁸, m, n, p, A, Q, T, W¹ und LG₂ jeweils wie oben definiert sind, und
optional eine oder alle der Verbindungen der Formeln VII, VI, V und III, wobei R⁸ Wasserstoff, Hydroxy, Chlor, Brom, Jod, C₁-C₆-Alkoxy oder C₃-C₈-Cycloalkoxy ist/sind in die Verbindung der Formeln VII, VI, V bzw. III umgewandelt, wobei R⁸ NR^{1a}R^{1b} ist und R^{1a} und R^{1b} jeweils wie oben definiert sind.

2. Prozess nach Anspruch 1,
wobei
R⁸ ausgewählt ist aus der Gruppe, bestehend aus Hydroxy, Chlor, C₁-C₆-Alkoxy oder NR^{1a}R^{1b},
wobei R^{1a} und R^{1b} unabhängig voneinander ausgewählt sind aus der Gruppe, bestehend aus Wasserstoff, C₁-C₆-Alkyl, (C₁-C₆-Alkyl)-C₃-C₆-Cycloalkyl und (C₃-C₆-Cycloalkyl)-C₁-C₆-Alkyl;
W¹ O ist; T Phenyl ist; R² ausgewählt ist aus der Gruppe, bestehend aus Wasserstoff, Halogen, Cyano und C₁-C₆-Alkyl; A N ist; R³ ausgewählt ist aus der Gruppe, bestehend aus Wasserstoff, Halogen und C₁-C₆-Halogenalkyl; R⁴ ausgewählt ist aus der Gruppe, bestehend aus Wasserstoff oder Halogen; Q ein 5-gliedriger heterocyclischer Ring ist; n eine ganze Zahl 1 ist; m eine ganze Zahl 2 ist; p eine ganze Zahl 1 oder 2 ist;
LG₂ ausgewählt ist aus der Gruppe, bestehend aus Chlor, Brom oder OR¹²,
wobei R¹² C₁-C₆-Alkyl ist;
LG₄ ausgewählt ist aus der Gruppe, bestehend aus Chlor oder OR¹³,
wobei R¹³ ausgewählt ist aus der Gruppe, bestehend aus Wasserstoff oder C₁-C₆-Alkyl; und
LG₁ ausgewählt ist aus der Gruppe, bestehend aus Chlor oder Brom; und LG₃ ein Alkalimetall ist, ausgewählt aus Natrium, Kalium und Lithium.

3. Prozess nach Anspruch 1,
wobei
R⁸ NR^{1a}R^{1b} ist, wobei R^{1a} Wasserstoff ist; und R^{1b} C₁-C₆-Alkyl, (C₁-C₆-Alkyl)-C₃-C₆-Cycloalkyl und (C₃-C₆-Cycloalkyl)-C₁-C₆-Alkyl ist; W¹ O ist; T Phenyl ist; R² ausgewählt ist aus der Gruppe, bestehend aus Halogen, Cyano und C₁-C₆-Alkyl; A N ist; R³ C₁-C₆-Halogenalkyl ist; R⁴ Halogen ist; Q ein Tetrazolylring ist; n eine ganze Zahl 1 ist; m eine ganze Zahl 2 ist; p eine ganze Zahl 1 ist; Ig₂ or¹² ist, wobei R¹² C₁-C₃-Alkyl ist; LG₄ Chlor ist; LG₁ Brom ist; und LG₃ Natrium ist.

4. Prozess nach Anspruch 1,
wobei
R⁸ NR^{1a}R^{1b} ist, wobei R^{1a} Wasserstoff ist; und R^{1b} Methyl oder 1-Cyclopropylethyl ist; W¹ O ist; T Phenyl ist; R² 4-Cyano und 2-Methyl, oder 2-Brom und 4-Chlor ist; A N ist; R³ 5-Trifluormethyl ist; R⁴ 3-Chlor ist; Q ein 2-Tetrazolylring ist; n eine ganze Zahl 1 ist; m eine ganze Zahl 2 ist; p eine ganze Zahl 1 ist; LG₂ Methoxy ist; LG₄ Chlor ist; LG₁ Brom ist; und LG₃ Natrium ist.

5. Prozess nach Anspruch 1, wobei der Prozess eine Vorstufe der Gewinnung der Verbindung der Formel VIII durch Umsetzung einer Verbindung der Formel IX und einer Verbindung der Formel X in einem geeigneten Lösungsmittel und optional einem geeigneten Reagenz umfasst, 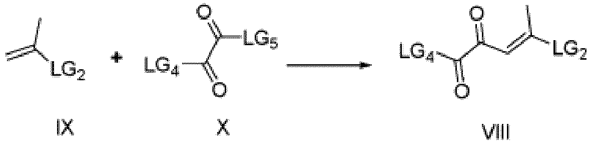 wobei
LG₅ Hydroxy oder Halogen ist; und LG₂ und LG₄ jeweils nach Anspruch 1 definiert sind;
das geeignete Lösungsmittel ausgewählt ist aus der Gruppe, bestehend aus Petrolether, n-Hexan, n-Heptan, Cyclohexan, Methylcyclohexan, Benzol, Toluol, Xylol, Decalin, Chlorbenzol, Dichlorbenzol, Dichlormethan, Chloroform, Tetrachlormethan, Dichlorethan, Trichlorethan, Diethylether, Diisopropylether, Methyl-tert-butylether, Methyl-tert-amylether, Dioxan, Tetrahydrofuran, 2-Methyl-Tetrahydrofuran, 1,2-Dimethoxyethan, 1,2-Diethoxyethan, Cyclopentylmethylether, Anisol, Acetonitril, Propionitril, n-Butyronitril, Iso-Butyronitril, Benzonitril oder Kombinationen davon;
das geeignete Reagenz ausgewählt ist aus der Gruppe, bestehend aus Pyridin, 1- oder 2- oder 3-Picolin, Triethylamin, *N,N*-Diisopropylethylamin, 2,6-Di-tert-butylpyridin, 1,5-Diazabicyclo(4.3.0)non-5-en, 1,8-Diazabicycloundec-7-en, Lithiumdiisopropylamid, Natriumbis(trimethylsilyl)amid und Kaliumbis(trimethylsilyl)amid oder Kombinationen davon; und
die Verbindung der Formel VIII isoliert werden kann oder nicht.

6. Prozess nach Anspruch 1, wobei das geeignete Lösungsmittel in den Schritten (a), (b), (c) und (d) ausgewählt ist aus der Gruppe, bestehend aus aliphatischen, alizyklischen oder aromatischen Kohlenwasserstoffen, halogenierten Kohlenwasserstoffen, Ethern, Nitrilen, Amiden, Alkoholen oder Kombinationen davon.

7. Prozess nach Anspruch 1, wobei das geeignete Lösungsmittel in den Schritten (a), (b), (c) und (d) ausgewählt ist aus der Gruppe, bestehend aus Petrolether, n-Hexan, n-Heptan, Cyclohexan, Methylcyclohexan, Benzol, Toluol, Xylol, Decalin, Chlorbenzol, Dichlorbenzol, Dichlormethan, Chloroform, Tetrachlormethan, Dichlorethan, Trichlorethan, Diethylether, Diisopropylether, Methyl-tert-butylether, Methyl-tert-amylether, Dioxan, Tetrahydrofuran, 2-Methyltetrahydrofuran, 1,2-Dimethoxyethan, 1,2-Diethoxyethan, Cyclopentylmethylether, Anisol, Acetonitril, Propionitril, n-Butyronitril, iso-Butyronitril, Benzonitril, N,N-Dimethylformamid, N,N-Dimethylacetamid, N-Methylformanilid, N-Methylpyrrolidon, Hexan-1-ethylphosphoramid, Dimethylsulfoxid, Sulfolan, Methanol, Ethanol, Isopropanol oder Kombinationen davon.

8. Prozess nach Anspruch 1, wobei
a) der Schritt (a) bei einer Temperatur im Bereich von 0 bis 120 °C durchgeführt wird; und
b) das geeignete Kupplungsreagenz in Schritt (a) ausgewählt ist aus der Gruppe, bestehend aus Pyridin, 1- oder 2- oder 3-Picolin, Triethylamin, N,N-Diisopropylethylamin, 2,6-Di-tert-butylpyridin, 1,5-Diazabicyclo(4.3.0)non-5-en, 1,8-Diazabicycloundec-7-en, Lithiumdiisopropylamid, Natriumbis(trimethylsilyl)amid und Kaliumbis(trimethylsilyl)amid oder Kombinationen davon.

9. Prozess nach Anspruch 1, wobei
a) der Schritt (b) bei einer Temperatur im Bereich von 0 bis 120 °C durchgeführt wird; und
b) das geeignete halogenierende Mittel in Schritt (b) ausgewählt ist aus der Gruppe, bestehend aus N-Chlorsuccinimid, N-Bromsuccinimid und N-lodsuccinimid, N-Chlorsaccharin, N-Bromsaccharin und N-Iodsaccharin, N-Chlorhydantoin, N-Bromhydantoin und N-Iodhydantoin, Cl₂, Br₂ oder I₂; und
c) der Radikalinitiator in Schritt (b) ausgewählt ist aus der Gruppe, bestehend aus *hv,* Dibenzoylperoxid, Wasserstoffperoxid, Di(n-propyl)peroxydicarbonat, t-Butylperoxybenzoat, Methylethylketonperoxid, 2,5-Dimethyl-2,5-di(t-butylperoxy)-3-hexyne, Di(t-butyl)peroxid, Acetonperoxid, Dicumylperoxid, Azobisisobutyronitril, Bis(2-ethylhexyl)peroxydicarbonat, (Peroxybis(propan-2,2-diyl))dibenzen, Peressigsäure, Metachlorperbenzoesäure, Paynes Reagenz, Magnesiummonoperphthalat, Trifluroperessigsäure, Trichlorperessigsäure, 2,4-Dinitorperbenzoesäure, Carosche Säure und Kaliumcaroat.

10. Prozess nach Anspruch 1, wobei der Schritt (c) bei einer Temperatur im Bereich von 15 bis 150 °C durchgeführt wird.

11. Prozess nach Anspruch 1, wobei
a) der Schritt (d) bei einer Temperatur im Bereich von 15 bis 150 °C durchgeführt wird; und
b) das geeignete dehydrierende Mittel in Schritt (d) ausgewählt ist aus der Gruppe, bestehend aus Salzsäure, Schwefelsäure, Orthoameisensäure, Phorphorpentoxid, Phosphorylchlorid, Calciumoxid, Eisen(III)-chlorid, N,N'-Dicyclohexylcarbodiimid, Cyanurchlorid und Burgess-Reagenz.

12. Verwendung einer Verbindung der Formel VIII, 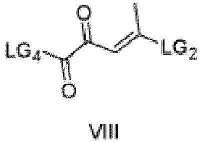 wobei LG₂ C₁-C₆-Alkoxy oder Halogen ausgewählt aus der Gruppe, bestehend aus Iodid, Chlorid und Bromid, ist; und LG₄ ein Halogen ist, das aus der Gruppe ausgewählt ist, bestehend aus Iodid, Chlorid und Bromid, als Zwischenprodukt in dem Prozess für die Synthese einer Verbindung der Formel I, wie nach Anspruch 1 beansprucht.

13. Verbindung der Formel XIII, 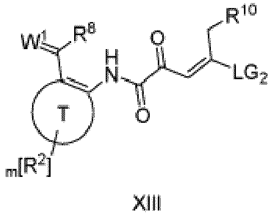 wobei
R¹⁰ Wasserstoff, LG₁ oder 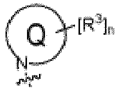 ist;
wobei
LG₁ ausgewählt ist aus der Gruppe, bestehend aus Fluor, Chlor, Brom oder Jod,
R³ ausgewählt ist aus der Gruppe, bestehend aus Wasserstoff, Halogen, Cyano, Nitro, Hydroxy, C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Haloalkoxy, C₁-C₆-Alkylthio, C₁-C₆-Alkylsulphinyl, C₁-C₆-Alkylsulphonyl, C₁-C₆-Haloalkylthio, C₁-C₆-Haloalkylsulfinyl und C₁-C₆-Haloalkylsulphonyl,
Q ein 3-, 4- oder 5-gliedriger heterocyclischer Ring ist und
n eine ganze Zahl von 0 bis 4 ist;
R⁸ ausgewählt ist aus der Gruppe, bestehend aus Wasserstoff, Hydroxy, Chlor, Brom, Jod, C₁-C₆-Alkoxy, C₃-C₈-Cy_{C}loalkoxy oder NR^{1a}R^{1b};
wobei
R^{1a} und R^{1b} unabhängig voneinander ausgewählt sind aus der Gruppe, bestehend aus Wasserstoff, C₁-C₆-Alkyl, C₁-C₆-Halogenalkyl, C₃-G₆-Cycloalkyl, (C₁-C₆-Alkyl)-C₃-C₆-Cycloalkyl und (C₃-C₆-Cycloalkyl)-C₁-C₆-Alkyl oder
R^{1a} und R^{1b} zusammen mit dem N-Atom, an das sie gebunden sind, N=S(=O)₀₋₂(C₁-C₆-Alkyl)₂ oder einen 3- oder 4- oder 5- oder 6-gliedrigen heterocyclischen Ring bilden, der ein oder mehrere Heteroatome, ausgewählt aus N, O und S, umfasst; und das C-Atom des heterocyclischen Rings durch C(=O), C(=S) und C(=NR⁷) ersetzt sein kann,
wobei
der heterocyclische Ring mindestens mit einer Gruppe substituiert sein kann, die ausgewählt ist aus der Gruppe, die besteht aus Halogen, Cyano, Nitro, Hydroxy, C₁-C₆-Alkyl, C₁-C₆-Haloalkyl, C₃-C₆-Cycloalkyl, C₃-C₆-Halocycloalkyl, C₁-C₆-Alkoxy, C₁-C₆-Haloalkoxy, C₁-C₆-Alkylthio, C₁-C₆-Alkylsulphinyl, C₁-C₆-Alkylsulphonyl, C₁-C₆-Haloalkylthio, C₁-C₆-Haloalkylsulphinyl, C₁-C₆-Haloalkylsulphonyl und
R⁷ ausgewählt ist aus der Gruppe, bestehend aus Wasserstoff, Hydroxy, Cyano, C₁-C₆-Alkyl, C₁-C₆-Haloalkyl, C₁-C₆-Alkoxy, C₁-C₆-Haloalkoxy, C₃-C₆-Cycloalkyl, C₃-C₆-Halocycloalkyl, C₃-C₆-Cycloalkoxy, C₃-C₆-Halocycloalkoxy;
W¹ ausgewählt ist aus O, S oder NR⁵;
R⁵ ausgewählt ist aus der Gruppe, bestehend aus Wasserstoff, Hydroxy, Cyano, Nitro, Halogen, C₁-C₆-Alkyl, C₁-C₆-Haloalkyl, C₁-C₆-Alkoxy, C₁-C₆-Haloalkoxy, C₃-C₆-Cycloalkyl, C₃-C₆-Halocycloalkyl;
oder
W¹ und R⁸ zusammen mit dem Kohlenstoffatom, an das sie gebunden sind, einen Oxazolinring 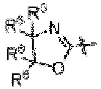 bilden können,
wobei 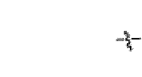
den Befestigungspunkt von T anzeigt; und
R⁶ unabhängig Wasserstoff oder C₁-C₆-Alkyl ist; und
Aryl- oder Heteroarylring oder -ringsystem;
R² ausgewählt ist aus der Gruppe, bestehend aus Wasserstoff, Halogen, Cyano, Nitro, C₁-C₆-Alkyl, C₁-C₆-Halogenalkyl und C₃-C₆-Cycloalkyl;
m eine ganze Zahl 2 ist; und
LG₂ C₁-C₆-Alkoxy oder Halogen ausgewählt aus der Gruppe, bestehend aus Iodid, Chlorid und Bromid, ist.

14. Verbindung nach Anspruch 13,
wobei
R¹⁰ Wasserstoff, LG₁ oder 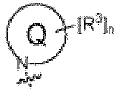 ist;
wobei
LG₁ Chlor oder Brom ist,
R³ C₁-C₆-Halogenalkyl ist,
Q ein 5-gliedriger heterocyclischer Ring ist und
n eine ganze Zahl 1 ist;
R⁸ ausgewählt ist aus der Gruppe, bestehend aus Hydroxy, Chlor, C₁-C₆-Alkoxy oder NR^{1a}R^{1b};
wobei
R^{1a} Wasserstoff ist und
R^{1b} C₁-C₆-Alkyl, (C₁-C₆-Alkyl)-C₃-C₆-Cycloalkyl oder (C₃-C₆-Cycloalkyl)-C₁-C₆-Alkyl ist;
W¹ ausgewählt ist aus O; T Phenyl ist; R² ausgewählt ist aus der Gruppe, bestehend aus Halogen, Cyano, C₁-C₆-Alkyl; m eine ganze Zahl 2 ist; und LG₂ Methoxy oder Ethoxy ist.

15. Verbindung nach Anspruch 13, wobei
R¹⁰ Wasserstoff, LG₁ oder 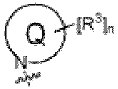 ist;
wobei
LG₁ Brom ist,
R³ 5-Trifluormethyl ist,
Q ein 2-Tetrazolylring ist und
n eine ganze Zahl 1 ist;
R⁸ NR^{1a}R^{1b} ist;
wobei
R^{1a} Wasserstoff ist und
R^{1b} Methyl oder 1-Cyclopropylethyl ist;
W¹ ausgewählt ist aus O; T Phenyl ist; R² 4-Cyano und 2-Methyl, oder 2-Brom und 4-Chlor ist; m eine ganze Zahl 2 ist; und LG₂ Methoxy ist.

## Revendications

1. Procédé pour la préparation d'un composé de formule I, 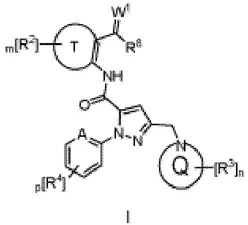 dans lequel,
R⁸ est choisi dans le groupe constitué d'hydrogène, hydroxy, chlore, brome, iode, alcoxy en C₁-C₆, cycloalcoxy en C₃-C₈ ou NR^{1a}R^{1b} ;
dans lequel,
R^{1a} et R^{1b} sont choisis indépendamment dans le groupe constitué d'hydrogène, alkyle en C₁-C₆, haloalkyle en C₁-C₆, cycloalkyle en C₃-C₆, cycloalkyl en C₃-C₆-alkyle en C₁-C₆ et alkyl en C₁-C₆-cycloalkyle en C₃-C₆, ou
R^{1a} et R^{1b} forment, conjointement avec l'atome N auquel ils sont attachés, N=S(=O)₀₋₂(alkyle en C₁-C₆)₂ ou un cycle hétérocyclique à 3 ou 4 ou 5 ou 6 membres comprenant un ou plusieurs hétéroatomes choisis parmi N, O et S ; et l'atome C du cycle hétérocyclique peut être remplacé par C(=O), C(=S) et C(=NR⁷),
dans lequel,
le cycle hétérocyclique peut être substitué par un ou plusieurs substituants choisis dans le groupe constitué d'halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆, haloalkylsulfonyle en C₁-C₆, et
R⁷ est choisi dans le groupe constitué d'hydrogène, cyano, alkyle en C₁-C₆, haloalkyle en C₁-C₆, alcoxy en C₁-C₆, haloalcoxy en C₁-C₆, cycloalkyle en C₃-C₆, halocycloalkyle en C₃-C₆, cycloalcoxy en C₃-C₆, halocycloalcoxy en C₃-C₆ ;
W¹ est choisi parmi O, S ou NR⁵,
dans lequel, R⁵ est choisi dans le groupe constitué d'hydrogène, hydroxy, cyano, nitro, halogène, alkyle en C₁-C₆, haloalkyle en C₁-C₆, alcoxy en C₁-C₆, haloalcoxy en C₁-C₆, cycloalkyle en C₃-C₆, halocycloalkyle en C₃-C₆ ;
ou
W¹ et R⁸, conjointement avec l'atome de carbone auquel ils sont attachés, peuvent former un cycle oxazoline 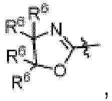
dans lequel, 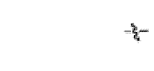
indique le point d'attache à T, et
R⁶ représente indépendamment l'hydrogène ou un alkyle en C₁-C₆ ;
un cycle ou système de cycles aryles ou hétéroaryles ;
R² est choisi dans le groupe constitué d'hydrogène, halogène, cyano, nitro, alkyle en C₁-C₆, haloalkyle en C₁-C₆ et cycloalkyle en C₃-C₆ ;
A représente N ou C ;
R³ est choisi dans le groupe constitué d'hydrogène, halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆ et haloalkylsulfonyle en C₁-C₆ ;
R⁴ est choisi dans le groupe constitué d'hydrogène, halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆, haloalkylsulfonyle en C₁-C₆ ou NR^{a}R^{b} ;
dans lequel, R^{a} et R^{b} sont indépendamment choisis dans le groupe constitué d'hydrogène, alkyle en C₁-C₆, cycloalkyle en C₃-C₆ ou R^{a} et R^{b} forment, conjointement avec l'atome N auquel ils sont attachés, un cycle hétérocyclique à 3 à 6 membres, substitué ou non substitué,
dans lequel, la substitution sur le cycle hétérocyclique à 3 à 6 membres est choisie dans le groupe constitué d'halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆ et haloalkylsulfonyle en C₁-C₆ ;
Q représente un cycle hétérocyclique à 3, 4 ou 5 membres ;
n représente un entier de 0 à 4 ;
m représente un entier de 0 à 6 ; et
p représente un entier de 0 à 5 ;
ledit procédé comprenant les étapes consistant à :
a) obtenir un composé de formule VI en faisant réagir un composé de formule VIII et un composé de formule VII dans un solvant approprié, en utilisant éventuellement un réactif de couplage approprié ; 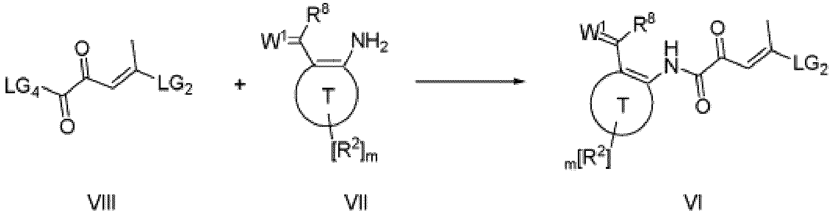
dans lequel,
LG₂ est choisi dans le groupe constitué de fluor, chlore, brome, iode ou OR¹², dans lequel, R¹² est choisi dans le groupe constitué d'hydrogène, alkyle en C₁-C₆ substitué ou non substitué, aryl-alkyle en C₁-C₆ substitué ou non substitué, aryle substitué ou non substitué, alkyle en -(C=O)-C₁-C₆, haloalkyle en -(C=O)-C₁-C₆, alkyle en -(C=O)O-C₁-C₆, alkyle en -(C=O)O-haloC₁-C₆, alkyle en SO₂-C₁-C₆, haloalkyle en SO₂-C₁-C₆, aryle en SO₂ substitué ou non et alkylthio ;
LG₄ est choisi dans le groupe constitué de chlore, brome, iode, N(OR¹³)R¹³, OR¹³ ou 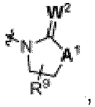
dans lequel, R¹³ est choisi dans le groupe constitué d'hydrogène, alkyle en C₁-C₆ substitué ou non substitué, aryl-alkyle en C₁-C₆ substitué ou non substitué, aryle substitué ou non substitué, alkyle en -(C=O)-C₁-C₆, haloalkyle en -(C=O)-C₁-C₆, alkyle en -(C=O)O-C₁-C₆, alkyle en -(C=O)O-haloC₁-C₆, alkyle en SO₂-C₁-C₆, haloalkyle en SO₂-C₁-C₆, aryle en SO₂ substitué ou non substitué et alkylthio,
dans lequel, les substitutions sur alkyle en C₁-C₆, aryl-alkyle en C₁-C₆, aryle et aryle en SO₂ de R¹² et R¹³ sont indépendamment choisies dans le groupe constitué d'halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆ et haloalkylsulfonyle en C₁-C₆,
W² et A¹ sont indépendamment choisis parmi O, S ou NR⁵, et
R⁹ est choisi dans le groupe constitué d'hydrogène, halogène, cyano, alkyle en C₁-C₆, haloalkyle en C₁-C₆, alcoxy en C₁-C₆, haloalcoxy en C₁-C₆, cycloalkyle en C₃-C₆, halocycloalkyle en C₃-C₆, alkylthio en C₁-C₆, haloalkylthio en C₁-C₆, alkylsulfinyle en C₁-C₆, haloalkylsulfinyle en C₁-C₆, alkylsulfonyle en C₁-C₆, haloalkylsulfonyle en C₁-C₆, aryle et arylalkyle ;
R², R⁵, R⁸, m, T et W¹ sont chacun tels que définis ci-dessus ;
b) ajouter des halogènes au composé de formule VI à l'aide d'un agent halogénant approprié dans un solvant approprié, éventuellement en présence d'un initiateur de radicaux, pour obtenir un composé de formule V ; 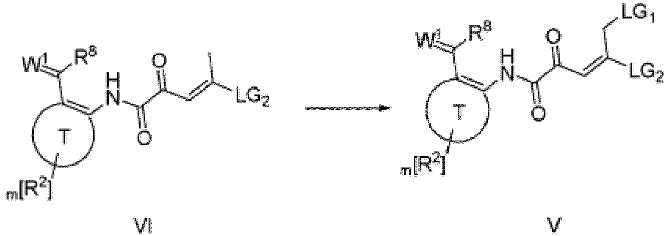
dans lequel,
LG₁ est choisi dans le groupe constitué de fluor, chlore, brome ou iode ; et
R², R⁸, m, T, W¹ et LG₂ sont chacun tels que définis ci-dessus,
c) faire réagir le composé de formule V et un composé de formule IV dans un solvant approprié pour obtenir un composé de formule III ; 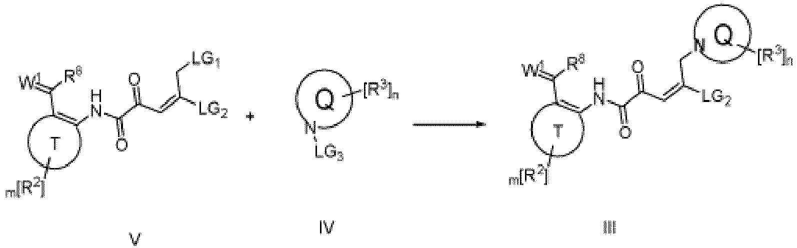
dans lequel,
LG₃ est choisi dans le groupe constitué d'hydrogène, halogène, Si(R¹¹)₃ et métal alcalin choisi parmi sodium, potassium et lithium,
dans lequel, R¹¹ est choisi dans le groupe constitué d'hydrogène, halogène, hydroxy, alkyle en C₁-C₆, haloalkyle en C₁-C₆, cycloalkyle en C₃-C₆, halocycloalkyle en C₃-C₆, alcoxy en C₁-C₆ et haloalcoxy en C₁-C₆, ;
R², R³, R⁸, m, n, Q, T, W¹, LG₁ et LG₂ sont chacun tels que définis ci-dessus ;
d) le composé de formule III est mis en réaction avec un composé de formule II dans un solvant approprié et un agent de déshydratation approprié pour obtenir le composé de formule I ; 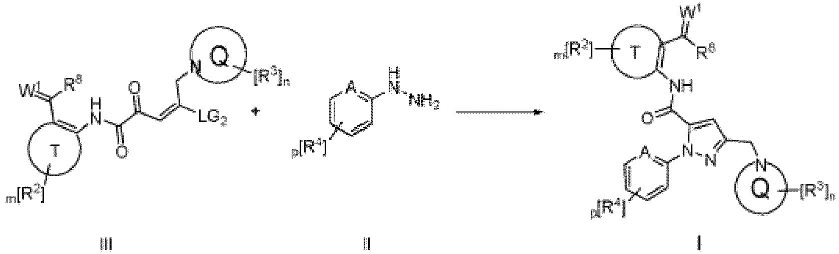
dans lequel,
les composés de formules VI, V et III peuvent être isolés ou non,
R², R³, R⁴, R⁸, m, n, p, A, Q, T, W¹ et LG₂ sont chacun tels que définis ci-dessus, et
éventuellement, tous les composés de formules VII, VI, V et III ou une partie de ceux-ci, dans lequel R⁸ représente hydrogène, hydroxy, chlore, brome, iode, alcoxy en C₁-C₆ ou cycloalcoxy en C₃-C₈, sont convertis en composé de formules VII, VI, V et III, respectivement, dans lequel R⁸ représente NR^{1a}R^{1b}, et R^{1a} et R^{1b} sont chacun tels que définis ci-dessus.

2. Procédé selon la revendication 1,
dans lequel,
R⁸ est choisi dans le groupe constitué d'hydroxy, chlore, alcoxy en C₁-C₆ ou NR^{1a}R^{1b},
dans lequel, R^{1a} et R^{1b} sont indépendamment choisis dans le groupe constitué d'hydrogène, alkyle en C₁-C₆, cycloalkyl en C₃-C₆-alkyle en C₁-C₆ et alkyl en C₁-C₆-cycloalkyle en C₃-C₆ ;
W¹ représente O ; T représente un phényle ; R² est choisi dans le groupe constitué d'hydrogène, halogène, cyano et alkyle en C₁-C₆ ; A représente N ; R³ est choisi dans le groupe constitué d'hydrogène, halogène et haloalkyle en C₁-C₆ ; R⁴ est choisi dans le groupe constitué d'hydrogène ou halogène ; Q représente un cycle hétérocyclique à 5 membres ; n représente un entier 1 ; m représente un entier 2 ; p représente un entier 1 ou 2 ;
LG₂ est choisi dans le groupe constitué de chlore, brome ou OR¹²,
dans lequel, R¹² représente un alkyle en C₁-C₆ ;
LG₄ est choisi dans le groupe constitué de chlore ou OR¹³,
dans lequel, R¹³ est choisi dans le groupe constitué d'hydrogène ou d'un alkyle en C₁-C₆ ; et
LG₁ est choisi dans le groupe constitué de chlore ou brome ; et LG₃ représente un métal alcalin choisi parmi sodium, potassium et lithium.

3. Procédé selon la revendication 1,
dans lequel,
R⁸ représente NR^{1a}R^{1b}, dans lequel, R^{1a} représente l'hydrogène ; et R^{1b} représente un alkyle en C₁-C₆, un cycloalkyl en C₃-C₆-alkyle en C₁-C₆ et un alkyl en C₁-C₆-cycloalkyle en C₃-C₆ ; W¹ représente O ; T représente un phényle ; R² est choisi dans le groupe constitué d'halogène, cyano et alkyle en C₁-C₆ ; A représente N ; R³ représente un haloalkyle en C₁-C₆ ; R⁴ représente un halogène ; Q représente un cycle tétrazolyle ; n représente un entier 1 ; m représente un entier 2 ; p représente un entier 1 ; LG₂ représente OR¹², dans lequel, R¹² représente un alkyle en C₁-C₃ ; LG₄ représente le chlore ; LG₁ représente le brome ; et LG₃ représente le sodium.

4. Procédé selon la revendication 1,
dans lequel,
R⁸ représente NR^{1a}R^{1b}, dans lequel, R^{1a} représente l'hydrogène ; et R^{1b} représente un méthyle ou le 1-cyclopropyléthyle ; W¹ représente O ; T représente un phényle ; R² représente 4-cyano et 2-méthyl, ou 2-bromo et 4-chloro ; A représente N ; R³ représente le 5-trifluoro méthyle ; R⁴ représente la 3-chlorine ; Q représente un cycle 2-tétrazolyle ; n représente un entier 1 ; m représente un entier 2 ; p représente un entier 1 ; LG₂ représente un méthoxy ; LG₄ représente le chlore ; LG₁ représente le brome ; et LG₃ représente le sodium.

5. Procédé selon la revendication 1, dans lequel ledit procédé comprend une étape préalable d'obtention du composé de formule VIII en faisant réagir un composé de formule IX et un composé de formule X dans un solvant approprié et éventuellement un réactif approprié, 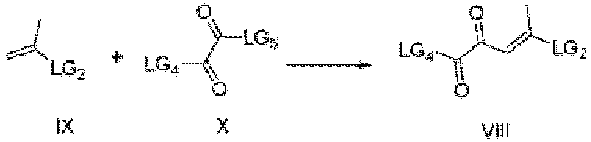 dans lequel,
LG₅ représente un hydroxy ou un halogène ; et LG₂ et LG₄ sont chacun tels que définis dans la revendication 1 ;
le solvant approprié est choisi dans le groupe constitué d'éther de pétrole, n-hexane, n-heptane, cyclohexane, méthylcyclohexane, benzène, toluène, xylène, décaline, chlorobenzène, dichlorobenzène, dichlorométhane, chloroforme, tétrachlorométhane, dichloroéthane, trichloroéthane, éther diéthylique, éther diisopropylique, méthyl tert-butyl éther, méthyl tert-amyl éther, dioxane, tétrahydrofurane, 2-méthyl tétrahydrofurane, 1,2-diméthoxyéthane, 1,2-diéthoxyéthane, cyclopentylméthyléther, anisole, acétonitrile, propionitrile, n-butyronitrile, iso-butyronitrile, benzonitrile ou des combinaisons de ceux-ci ;
le réactif approprié est choisi dans le groupe constitué de pyridine, 1-picoline ou 2-picoline ou 3-picoline, triéthylamine, *N,N*-diisopropyléthylamine, 2,6-di-tert-butylpyridine, 1,5-diazabicyclo(4.3.0)non-5-ène, 1,8-diazabicycloundéc-7-ène, diisopropylamide de lithium, bis(triméthylsilyl)amide de sodium et bis(triméthylsilyl)amide de potassium ou des combinaisons de ceux-ci ; et
le composé de formule VIII peut être isolé ou non.

6. Procédé selon la revendication 1, dans lequel le solvant approprié aux étapes (a), (b), (c) et (d) est choisi dans le groupe constitué d'hydrocarbures aliphatiques, alicycliques ou aromatiques, hydrocarbures halogénés, éthers, nitriles, amides, alcools ou des combinaisons de ceux-ci.

7. Procédé selon la revendication 1, dans lequel le solvant approprié aux étapes (a), (b), (c) et (d) est choisi dans le groupe constitué d'éther de pétrole, n-hexane, n-heptane, cyclohexane, méthylcyclohexane, benzène, toluène, xylène, décaline, chlorobenzène, dichlorobenzène, dichlorométhane, chloroforme, tétrachlorométhane, dichloroéthane, trichloroéthane, éther diéthylique, éther diisopropylique, méthyl tert-butyl éther, méthyl tert-amyl éther, dioxane, tétrahydrofurane, 2-méthyl tétrahydrofurane, 1,2-diméthoxyéthane, 1,2-diéthoxyéthane, cyclopentylméthyléther, anisole, acétonitrile, propionitrile, n-butyronitrile, iso-butyronitrile, benzonitrile, N,N-diméthylformamide, N,N-diméthylacétamide, N-méthylformanilide, N-méthylpyrrolidone, hexan-1-éthylphosphoranlide, diméthylsulfoxyde, sulfolane, méthanol, éthanol, isopropanol ou des combinaisons de ceux-ci.

8. Procédé selon la revendication 1, dans lequel
a) l'étape (a) est effectuée à une température allant de 0 à 120 °C ; et
b) le réactif de couplage approprié à l'étape (a) est choisi dans le groupe constitué de pyridine, 1-picoline ou 2-picoline ou 3-picoline, triéthylamine, *N,N-*diisopropyléthylamine, 2,6-di-tert-butylpyridine, 1,5-diazabicyclo(4.3.0)non-5-ène, 1,8-diazabicycloundéc-7-ène, diisopropylamide de lithium, bis(triméthylsilyl)amide de sodium et bis(triméthylsilyl)amide de potassium ou des combinaisons de ceux-ci.

9. Procédé selon la revendication 1, dans lequel
a) l'étape (b) est effectuée à une température allant de 0 à 120 °C ; et
b) l'agent halogénant approprié à l'étape (b) est choisi dans le groupe constitué de N-chlorosuccinimide, N-bromosuccinimide et N-iodosuccinimide, N-chlorosaccharine, N-bromosaccharine et N-iodosaccharine, N-chlorohydantoïne, N-bromohydantoïne et N-iodohydantoïne, Cl₂, Br₂ ou I₂ ; et
c) l'initiateur de radicaux à l'étape (b) est choisi dans le groupe constitué de *hv,* peroxyde de dibenzoyle, peroxyde d'hydrogène, di(n-propyl)peroxydicarbonate, peroxybenzoate de t-butyle, peroxyde de méthyléthylcétone, 2,5-diméthyl-2,5-di(t-butylperoxy)-3-hexyne, di(t-butyl)peroxyde, peroxyde d'acétone, peroxyde de dicumyle, azobisisobutyronitrile, bis(2-éthylhexyl)peroxydicarbonate, (peroxybis(propane-2,2-diyl))dibenzène, acide peracétique, acide métachloroperbenzoïque, réactif de Payne, monoperphtalate de magnésium, acide trifluoroperacétique, acide trichloroperacétique, acide 2,4-dinitroperbenzoïque, acide de Caro et caroate de potassium.

10. Procédé selon la revendication 1, dans lequel l'étape (c) est effectuée à une température allant de 15 à 150 °C.

11. Procédé selon la revendication 1, dans lequel
a) l'étape (d) est effectuée à une température allant de 15 à 150 °C ; et
b) l'agent de déshydratation approprié à l'étape (d) est choisi dans le groupe constitué d'acide chlorhydrique, acide sulfurique, acide orthoformique, pentoxyde de phosphore, chlorure de phosphoryle, oxyde de calcium, chlorure de fer(III), N,N'-dicyclohexylcarbodiimide, chlorure cyanurique et réactif de Burgess.

12. Utilisation d'un composé de formule VIII, 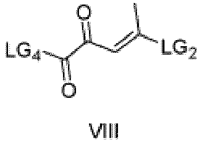 dans laquelle, LG₂ représente un alcoxy en C1-C6 ou un halogène choisi dans le groupe constitué d'iodure, chlorure et bromure ; et LG₄ représente un halogène choisi dans le groupe constitué d'iodure, chlorure et bromure, en tant qu'intermédiaire dans le procédé pour la synthèse d'un composé de formule I selon la revendication 1.

13. Composé de formule XIII, 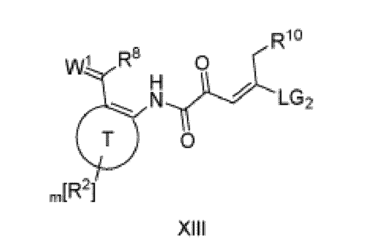 dans lequel,
R¹⁰ représente l'hydrogène, LG₁ ou 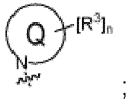
dans lequel,
LG₁ est choisi dans le groupe constitué de fluor, chlore, brome ou iode,
R³ est choisi dans le groupe constitué d'hydrogène, halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆ et haloalkylsulfonyle en C₁-C₆,
Q représente un cycle hétérocyclique à 3, 4 ou 5 membres, et
n représente un entier de 0 à 4 ;
R⁸ est choisi dans le groupe constitué d'hydrogène, hydroxy, chlore, brome, iode, alcoxy en C₁-C₆, cycloalcoxy en C₃-C₈ ou NR^{1a}R^{1b} ;
dans lequel,
R^{1a} et R^{1b} sont choisis indépendamment dans le groupe constitué d'hydrogène, alkyle en C₁-C₆, haloalkyle en C₁-C₆, cycloalkyle en C₃-C₆, cycloalkyl en C₃-C₆-alkyle en C₁-C₆ et alkyl en C₁-C₆-cycloalkyle en C₃-C₆ ;
R^{1a} et R^{1b} forment, conjointement avec l'atome N auquel ils sont attachés, N=S(=O)₀₋₂(alkyle en C₁-C₆)₂ ou un cycle hétérocyclique à 3 ou 4 ou 5 ou 6 membres comprenant un ou plusieurs hétéroatomes choisis parmi N, O et S ; et l'atome C du cycle hétérocyclique peut être remplacé par C(=O), C(=S) et C(=NR⁷),
dans lequel,
le cycle hétérocyclique peut être substitué par au moins un groupe choisi dans le groupe constitué d'halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆, haloalkylsulfonyle en C₁-C₆, et
R⁷ est choisi dans le groupe constitué d'hydrogène, hydroxy, cyano, alkyle en C₁-C₆, haloalkyle en C₁-C₆, alcoxy en C₁-C₆, haloalcoxy en C₁-C₆, cycloalkyle en C₃-C₆, halocycloalkyle en C₃-C₆, cycloalcoxy en C₃-C₆, halocycloalcoxy en C₃-C₆ ;
W¹ est choisi parmi O, S ou NR⁵ ;
R⁵ est choisi dans le groupe constitué d'hydrogène, hydroxy, cyano, nitro, halogène, alkyle en C₁-C₆, haloalkyle en C₁-C₆, alcoxy en C₁-C₆, haloalcoxy en C₁-C₆, cycloalkyle en C₃-C₆, halocycloalkyle en C₃-C₆ ;
ou
W¹ et R⁸, conjointement avec l'atome de carbone auquel ils sont attachés, peuvent former un cycle oxazoline 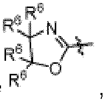
dans lequel, 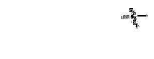
indique le point d'attache à T ; et
R⁶ représente indépendamment l'hydrogène ou un alkyle en C₁-C₆ ; et et
un cycle ou système de cycles aryles ou hétéroaryles ;
R² est choisi dans le groupe constitué d'hydrogène, halogène, cyano, nitro, alkyle en C₁-C₆, haloalkyle en C₁-C₆ et cycloalkyle en C₃-C₆ ;
m représente un entier 2 ; et
LG₂ représente un alcoxy en C₁-C₆ ou un halogène choisi dans le groupe constitué d'iodure, chlorure et bromure.

14. Composé selon la revendication 13, dans lequel,
R¹⁰ représente l'hydrogène, LG₁ ou 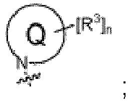
dans lequel,
LG₁ représente le chlore ou le brome,
R³ représente un haloalkyle en C₁-C₆,
Q représente un cycle hétérocyclique à 5 membres, et
n représente un entier 1 ;
R⁸ est choisi dans le groupe constitué d'hydroxy, chlore, alcoxy en C₁-C₆ ou NR^{1a}R^{1b} ;
dans lequel,
R^{1a} représente l'hydrogène, et
R^{1b} représente un alkyle en C₁-C₆, un cycloalkyl en C₃-C₆-alkyle en C₁-C₆ ou un alkyl en C₁-C₆-cycloalkyle en C₃-C₆ ;
W¹ est choisi parmi O ; T représente un phényle ; R² est choisi dans le groupe constitué d'halogène, cyano, alkyle en C₁-C₆ ; m représente un entier 2 ; et LG₂ représente un méthoxy ou un éthoxy.

15. Composé selon la revendication 13, dans lequel,
R¹⁰ représente l'hydrogène, LG₁ ou 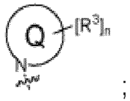
dans lequel,
LG₁ représente le brome,
R³ représente le 5-trifluoro méthyle,
Q représente un cycle 2-tétrazolyle, et
n représente un entier 1 ;
R⁸ représente NR^{1a}R^{1b} ;
dans lequel,
R^{1a} représente l'hydrogène, et
R^{1b} représente un méthyle ou le 1-cyclopropyléthyle ;
W¹ est choisi parmi O ; T représente un phényle ; R² représente 4-cyano et 2-méthyl, ou 2-bromo et 4-chloro ; m représente un entier 2 ; et LG₂ représente un méthoxy.
